# EUROPÄISCHE PATENTSCHRIFT

(11) **EP 4 255 363 B1**
(45) Veröffentlichungstag und Bekanntmachung des Hinweises auf die Patenterteilung: **18.02.2026**
(21) Anmeldenummer: 21807100.9
(22) Anmeldetag: 10.11.2021
(51) Int. Cl.: A61G 5/04, A61G 5/10

(54) **VORRICHTUNG UND VERFAHREN ZUM NAVIGIEREN UND/ODER BAHNFÜHREN EINES FAHRZEUGS UND FAHRZEUG**
DEVICE AND METHOD FOR NAVIGATING AND/OR GUIDING THE PATH OF A VEHICLE, AND VEHICLE
DISPOSITIF ET PROCÉDÉ POUR ASSURER LA NAVIGATION ET/OU LE GUIDAGE DE TRAJECTOIRE D'UN VÉHICULE ET VÉHICULE

(30) Priorität: 01.12.2020 DE 102020131845
(43) Veröffentlichungstag der Anmeldung: 11.10.2023
(73) Patentinhaber: Munevo GmbH, 80992 München (DE)
(72) Erfinder: HIDAS, Claudiu, 80805 München (DE); TRIVEDI, Aashish, 81369 München (DE); PANDEY, Deepesh, 80687 München (DE); MADAUS, Konstantin, 80639 München (DE)
(74) Vertreter: Meissner Bolte Partnerschaft mbB
(86) Internationale Anmeldenummer: PCT/EP2021/081242
(87) Internationale Veröffentlichungsnummer: WO 2022/117298

(56) Entgegenhaltungen:
- WO-A1-01/67537
- US-A- 4 407 393
- US-A1- 2017 042 439
- US-A1- 2017 266 069
- US-A1- 2020 222 219
- US-B1- 9 342 144

## Beschreibung

Die Erfindung betrifft eine Vorrichtung und ein Verfahren zum Navigieren und/oder Bahnführen eines Fahrzeugs, insbesondere Rollstuhls, sowie ein Fahrzeug.

US 4 407 393 legt einen Rollstuhl offen, welcher über Sensoren mittels Signalen gesteuert wird, die von einem Körperteil des Passagiers, nämlich dem Mund, ihren Ausgang nehmen.

Aus der EP 3 646 127 ist eine Rollstuhl-Sondersteuerung für einen elektrischen Rollstuhl bekannt. Diese umfasst ein Eingabeelement, beispielsweise eine am Kopf eines Passagiers angebrachte Datenbrille, sowie eine Adapterbox zur Übertragung der Daten des Eingabeelements an ein Input-/Output-Modul des elektrischen Rollstuhls. Zur Erzeugung von Fahrbefehlen werden bei Verwendung der Datenbrille Kopfbewegungen eines Passagiers von bewegungserfassenden Sensoren erfasst und gewünschten Fahrtrichtungen und Fahrtgeschwindigkeiten zugeordnet. Dem Input-/Output-Modul des Rollstuhls werden dabei basierend auf den Fahrbefehlen erzeugte Steuersignale zur Steuerung des Rollstuhls übermittelt.

Die EP 3 076 906 betrifft eine Steuereinheit für ein persönliches Fahrzeug. Die Steuereinheit ist in der Lage mit Hilfe zweier unabhängiger Bewegungssensoren ein Steuersignal für das Fahrzeug durch Auswerten der ermittelten relativen Orientierungen der unabhängigen Bewegungssensoren zueinander bereitzustellen. Dadurch kann auch bei Vorhandensein von Störgrößen, beispielsweise von Steigungen, Gefällen oder unbefestigten Straßen, ein gewünschter Fahrbefehl des Passagiers erkannt werden. Wenn die Auswertung der Orientierungssensoren bereits bekannte Gesten identifiziert, werden diese als Fahrzeug-Steuersignal verwendet. Zum Konfigurieren sowie zur Bestimmung von Position und Lage des Fahrzeugs können weitere, externe Sensoren verwendet werden.

Der beschriebene Stand der Technik ist in vielerlei Hinsicht problematisch. Zwar kann in vielen Fällen eine bewegungs- bzw. orientierungsbasierte Steuerung unter Verwendung von Daten mindestens eines ersten Bewegungssensors durchgeführt werden. Auch erlauben einige Ausführungsformen das Erkennen von Bewegungsmustern, um etwa einen Nothalt, Notruf oder das Starten externer Anwendungen einzuleiten. Ein Ausfall des ersten Bewegungssensors kann jedoch bereits zum Ausfall der Fahrzeugsteuerung führen. So führt bei einer Fahrzeugsteuerung, die rein auf Kopforientierungen basiert, bereits ein Krampf oder eine kurzfristig eintretende Versteifung des Genicks zum Verlust der Steuerfähigkeit durch den Passagier. Auch können vom Passagier nichtgewünschte Steuerbefehle, beispielsweise ausgelöst durch Fehler des ersten Bewegungssensors, an die Aktuatoren des Fahrzeugs weitergegeben werden und dieses in Bewegung versetzen oder belassen.

Ausgehend von diesem Stand der Technik ist es die Aufgabe der vorliegenden Erfindung eine Vorrichtung und ein Verfahren bereitzustellen, das erhöhte Sicherheit sowie erhöhten Komfort beim Betrieb eines Fahrzeugs schafft. Insbesondere soll eine erhöhte Ausfallsicherheit ermöglicht werden, die Übermittlung unerwünschter Steuerbefehle an das Fahrzeug soll vermieden werden und der Komfort bei Verwendung des Fahrzeugs soll verbessert werden. Die Aufgabe wird durch die Vorrichtung nach Anspruch 1, das Fahrzeug nach Anspruch 14 und das Verfahren nach Anspruch 15 gelöst.

Die Aufgabe wird insbesondere gelöst durch eine Vorrichtung zum Navigieren und/oder Bahnführen eines Fahrzeugs, insbesondere eines Rollstuhls, wobei die Vorrichtung zumindest umfasst:
- einen ersten Sensor, insbesondere einen Trägheitsnavigationssensor, der dazu ausgebildet und angeordnet ist, mindestens ein erstes Körperteil, insbesondere eine Lage und/oder Position und/oder Rotation und/oder Translation, eines Passagiers des Fahrzeugs zu erfassen und erste Sensorsignale auszugeben.
- einen zweiten Sensor, insbesondere bildbasierten Sensor, der dazu ausgebildet und angeordnet ist, mindestens das erste Körperteil des Passagiers und/oder dessen Merkmale, insbesondere deren Lage und/oder Position und/oder Rotation und/oder Translation, zu erfassen und zweite Sensorsignale auszugeben.
- eine Steuereinheit, die dazu ausgebildet ist, die ersten und zweiten Sensorsignale zu empfangen, basierend zumindest auf den ersten Sensorsignalen erste Steuersignale zur Steuerung des Fahrzeugs zu ermitteln, basierend zumindest auf den zweiten Sensorsignalen zu ermitteln, ob die ersten Steuersignale mindestens erste Verlässlichkeitskriterien erfüllen und Einnehmen eines Sicherheitsmodus, wenn die Steuereinheit bestimmt, dass die ersten Steuersignale nicht mindestens die ersten Verlässlichkeitskriterien erfüllen.

Ein Gedanke der Erfindung ergibt sich durch die Tatsache, dass jeder Sensor einer nicht vollständig eliminierbaren Fehler- oder Ausfallrate unterliegt. Dabei ist es insbesondere bei Anwendungen mit signifikanten, negativen Auswirkungen eines Sensorausfalls vorteilhaft, geeignete Maßnahmen für den Umgang mit Sensorfehlern bereitzustellen. Die vorliegende Erfindung nimmt sich diesem Umstand an, indem erste Sensorsignale von mindestens einem ersten Sensor nicht unmittelbar zum Ermitteln eines Steuersignals für das Fahrzeug Verwendung finden, sondern mindestens zweite Sensorsignale, insbesondere bildbasierte Sensorsignale, eines zweiten Sensors dazu verwendet werden mindestens die ersten Verlässlichkeitskriterien zu ermitteln, sodass ein Sicherheitsmodus eingenommen wird, wenn mindestens erste unter Verwendung des ersten Sensorsignals ermittelte Steuersignale die ersten Verlässlichkeitskriterien nicht erfüllen.

Als Sicherheitsmodus kann ein eingeschränkter Betriebsbereich der Vorrichtung verstanden werden, wobei der Einschränkungsumfang einerseits basierend auf sensorisch erfassbaren, körperlichen Zuständen eines Passagiers festgelegt wird. Andererseits können auch verringerte Genauigkeit und/oder Zuverlässigkeit der von der Vorrichtung verwendeten Sensoren und Aktoren zu einer Einschränkung des Betriebsbereichs der Vorrichtung führen, z. B. Fahren bei verringerter Geschwindigkeit.

Die Verlässlichkeitskriterien sind dazu bestimmt Schwellwerte festzulegen, ab denen der Sicherheitsmodus, d. h. der eingeschränkte Betriebsbereich der Vorrichtung, eingenommen werden muss. In Bezug auf die erfassbaren, körperlichen Zustände des Passagiers können die Schwellwerte allgemeine Schwellwerte oder individualisierte Schwellwerte sein. Individualisierte Schwellwerte können notwendig werden, wenn die äußerlich wahrnehmbaren Ausdrucksformen körperlicher Zustände, Mimik oder Gestik, eines Passagiers bedeutend eingeschränkt sind. Dies kann beispielsweise bei (Teil-)Lähmung eines Körperteils, beispielsweise des Gesichts, der Fall sein. Dabei ist es möglich, dass Schwellwerte durch die Vorrichtung durch mehrmalige Wiederholung bestimmter Mimiken oder Gestiken gelernt werden. Auch kann eine Anpassung der Schwellwerte während des Betriebs der Vorrichtung geschehen, beispielsweise wenn bekannt ist, dass ein Passagier bei längerem Betrieb des Fahrzeugs infolge der allgemeinen Anstrengung gewöhnlich zu zittern beginnt.

Durch die Verlässlichkeitskriterien können auch Schwellwerte für die notwendige Genauigkeit und/oder Zuverlässigkeit einzelner Sensoren definiert sein. Zudem können zulässige Toleranzen in den Abweichungen gleicher physikalischer Parameter, ermittelt von verschiedenen Sensoren, definiert werden. Ein Beispiel hierfür ist die zulässige Positionsabweichung der Iris bei einer augenmerkmalbasierten Steuerung des Fahrzeugs, bei welcher zwei unterschiedliche Sensoren das Auge des Passagiers erfassen.

Unter Verwendung mindestens der zweiten Sensorsignale können Aussagen darüber getroffen werden, ob sich der Passagier in der Lage befindet, das Fahrzeug zu steuern. Wie darüber zu entscheiden ist, ob ein Passagier zur Steuerung des Fahrzeugs in der Lage ist hängt von der gewählten Ausführungsform der vorliegenden Erfindung ab. Es ist möglich durch Vergleich der zweiten Sensorsignale mit vordefinierten, ersten Verlässlichkeitskriterien zu ermitteln, ob der Passagier sich in der Lage befindet das Fahrzeug zu steuern.

So kann beispielsweise bei einem Passagier durch mindestens den zweiten Sensor eine starke Neigung des Kopfs auf eine Seite über einen längeren Zeitraum infolge progressiv nachlassender Halsmuskelkraft erfasst werden. Bei angewandter kopforientierungsbasierter Steuerung könnte dies zu einer potentiell gefährlichen Kreisbewegungsfahrt führen. In einem solchen Fall kann der Sicherheitsmodus den zulässigen Betriebsbereich der Vorrichtung so anpassen, dass das Fahrzeug bis in den Stillstand abbremst, indem ein zweites Steuersignal ausgegeben wird.

Das Abbremsen in den Stillstand ist eine mögliche, fahrdynamikbeeinflussende, Maßnahme in Reaktion auf sensorisch erfassbare körperliche Zustände.

Auch ist es möglich vor dem Anhalten des Fahrzeugs eine sichere Anhalteposition anhand erweiterter Sensoren zur Positionsbestimmung oder einer umgebungserfassenden Sensorbaugruppe zu ermitteln und diese Anhalteposition anzusteuern. Auch hierbei werden von der Vorrichtung zweite Steuersignale generiert, die ungleich den ersten Steuersignalen sind.

Bei den ersten bzw. zweiten Steuersignalen kann es sich um fahrdynamikbeeinflussende Steuersignale handeln, die an Fahrzeug-Aktuatoren übermittelt werden. Es ist erfindungsgemäß zudem möglich, dass die Steuersignale Daten zur Vorrichtungs- bzw. Passagierüberwachung oder Wahlmöglichkeiten für einzunehmende Fahrzeugmodi an ein tragbares Computersystem (Wearable) oder ein Mobiltelefon des Passagiers bereitstellen. Auch können die Steuersignale für eine Weiterleitung an ein entfernt angeordnetes Computersystem (Backend) zur Vorrichtungs- bzw. Passagierüberwachung aus der Entfernung ausgebildet sein.

Die Vorrichtung erlaubt im Sicherheitsmodus den Betriebsbereich so anzupassen, dass ein Notrufmodus eingeleitet wird. Dadurch können vorbestimmte Personen vom eingeleiteten Sicherheitsmodus informiert werden. Bei Bedarf können auch die erfassten körperlichen Zustände des Passagiers übermittelt werden, beispielsweise um eine menschliche Diagnose der körperlichen Zustände des Passagiers aus der Entfernung durchzuführen.

Auch kann ein Sicherheitsmodus aufgrund nicht ausreichender Sensorbefähigung eingeleitet werden. Es ist denkbar, dass einer oder mehrere der Sensoren der Steuereinheit eigene Genauigkeits- oder Zuverlässigkeitsanalysedaten bereitstellen, woraufhin der eine oder mehrere Sensoren nicht für die Ermittlung der ersten Steuersignale oder der Verlässlichkeitskriterien Verwendung finden dürfen.

Wenn von der Vorrichtung als valide eingestufte erste Steuersignale oder zweite Steuersignale generiert werden, werden diese an Fahrzeug-Aktuatoren, insbesondere ansteuerbare Motoren, übermittelt, wobei die Fahrzeug-Aktuatoren zum Antreiben des Fahrzeugs angeordnet und zum Empfangen und Verarbeiten der Steuersignale der Vorrichtung ausgebildet sind.

Erfindungsgemäß kann ein erstes Körperteil ein beliebiges Körperteil des menschlichen Körpers sein, das in der Lage ist Bewegungen auszuführen.

In einer Ausführungsform handelt es sich beim ersten Körperteil um den Kopf des Passagiers. In dieser Ausführungsform umfasst die Vorrichtung als ersten Sensor eine Trägheitsnavigationseinheit, angeordnet an oder in dem tragbaren Computersystem (Wearable), die dazu ausgebildet ist, mindestens den Kopf, insbesondere eine Lage und/oder Position und/oder Rotation und/oder Translation, zu erfassen und erste Sensorsignale auszugeben.

Der den Kopf des Passagiers erfassende zweite Sensor kann ein bildbasierter Sensor, beispielsweise ein bildbasierter Frontsensor oder ein bildbasierter Rückseitensensor eines Mobiltelefons sein. Es kann sich jedoch auch um einen am Fahrzeug angebrachten, zusätzlichen bildbasierten Sensor handeln, der den Kopf des Passagiers erfasst. Die Steuereinheit kann dazu ausgebildet sein, die ersten und zweiten Sensorsignale zu empfangen und basierend auf den ersten Sensorsignalen erste Steuersignale zur Steuerung des Fahrzeugs zu ermitteln.

Es kann basierend auf den zweiten Sensorsignalen ermittelt werden, ob die ersten Steuersignale mindestens erste Verlässlichkeitskriterien erfüllen. Dazu werden die ersten Verlässlichkeitskriterien basierend auf den zweiten Sensorsignalen ermittelt. Der Sicherheitsmodus kann eingenommen werden, wenn die Steuereinheit bestimmt, dass die ersten Steuersignale nicht mindestens die ersten Verlässlichkeitskriterien erfüllen.

Anhand der zweiten Sensorsignale können dabei Basisdaten ermittelt werden. Diese Basisdaten können geometrische Abmessungen und/oder Größenverhältnisse und/oder Merkmale des Gesichts und/oder Gesten sein, wobei unter Verwendung der Basisdaten die ersten Verlässlichkeitskriterien ermittelt werden.

Diese geometrisch bzw. mathematisch ausgedrückten Eigenschaften der Basisdaten können die von Menschen wahrnehmbaren Gesichtsausdrücke charakterisieren und erlauben Rückschlüsse auf die vom Passagier empfundene Gefühlslage während des Betriebs des Fahrzeugs. Einerseits sollen die ersten Verlässlichkeitskriterien bei hoher Wahrscheinlichkeit eines Glückszustands, eines Entspannungszustands oder eines ähnlichen, mit positiver Stimmung assoziierten Zustands des Passagiers ein Erfüllen der ersten Verlässlichkeitskriterien bedeuten. Andererseits sollen die ersten Verlässlichkeitskriterien bei hoher Wahrscheinlichkeit von Besorgnis, eines Überraschungszustands, eines Angstzustands oder eines ähnlichen, mit negativer Stimmung assoziierten Zustands des Passagiers ein Nichterfüllen der ersten Verlässlichkeitskriterien bedeuten.

Erfindungsgemäß können durch die bildbasierte Analyse auch Rötungen, Vergrößerungen oder Verkleinerungen der Augen, inklusive der Pupillen sowie das Öffnen oder Schließen der Augen oder des Mundes und der Lippen inklusive ableitbarer Eigenschaften wie Kurzatmigkeit, erhöhter Puls oder zunehmende, gefährliche, körperliche Anstrengung der Person erfasst werden. Ein Erkennen derartiger körperliche Zustände bedeutet ebenfalls das Nichterfüllen der ersten Verlässlichkeitskriterien.

Zudem können erste Verlässlichkeitskriterien nicht erfüllt sein, wenn die ersten Steuersignale mit mindestens den zweiten Sensorsignalen in Widerspruch stehende Steuerbefehle des Passagiers erfassen, also der eine Sensor beispielsweise eine gewünschte Fahrt nach links, mindestens ein anderer Sensor eine gewünschte Fahrt nach rechts ermittelt.

Wenn die ersten Verlässlichkeitskriterien nicht erfüllt werden, wird der Sicherheitsmodus eingeleitet. Erfindungsgemäß ist es folglich möglich, ein bereits generiertes Steuersignal aufgrund des Verletzens erster Verlässlichkeitskriterien nicht an für die Fahrzeugsteuerung zuständige Fahrzeug-Aktuatoren zu übermitteln. Durch diese Anordnung kann gewährleistet werden, dass offensichtlich erkennbare, kritische körperliche Zustände des Passagiers erfasst werden, das Fahrzeug angehalten wird und/oder ein dem Sicherheitsmodus nachgelagerter Notrufmodus schnelle Hilfe für den Passagier durch Kontaktierung eines Notfallkontakts ermöglicht. Zudem können Dritte sowie der Passagier in der Folge nicht weiter körperlich geschädigt werden.

In einer Ausführungsform ist die Steuereinheit (ferner) ausgebildet, neben den ersten Sensorsignalen der an oder im Wearable angebrachten Trägheitsnavigationseinheit unter Verwendung auch der zweiten Sensorsignale des bildbasierten Sensors die ersten Steuersignale zu ermitteln. Dabei wird der Sicherheitsmodus eingenommen, wenn die ersten oder die zweiten Verlässlichkeitskriterien nicht erfüllt werden. In dieser Ausführungsform basieren die zweiten Verlässlichkeitskriterien auf Genauigkeits- und/oder Zuverlässigkeitsanalyse mindestens der ersten und der zweiten Sensorsignale.

Die Genauigkeits- und/oder Zuverlässigkeitsanalyse kann unter Verwendung von mit den Sensorsignalen bereitgestellten Selbstdiagnosedaten der Sensoren durchgeführt werden.

Die Genauigkeits- und/oder Zuverlässigkeitsanalyse kann zudem unter Verwendung jeweils der ersten und der zweiten Sensorsignale einer oder mehrerer der Merkmale Lage und/oder Position und/oder Rotation und/oder Translation des Kopfs des Passagiers ermittelt werden und anhand deren Abgleich die Zuverlässigkeit der beiden Sensoren abgeschätzt werden.

Wenn der Abgleich des für die Steuerung des Fahrzeugs verwendeten Merkmals bzw. der Merkmale innerhalb einer vordefinierten Toleranz bzw. vordefinierter Toleranzen übereinstimmt, werden für das Ermitteln des ersten Steuersignals die Merkmalswerte der Trägheitsnavigationseinheit verwendet. Sollten eine oder mehrere der Toleranzen überschritten werden, wird auf die Selbstdiagnosedaten des bildbasierten Sensors zurückgegriffen und bei schlechter Zuverlässigkeit, beispielsweise aufgrund von schlechter Bildqualität, ein Signal über die schlechte Zuverlässigkeit dem Passagier über das Wearable oder auf sein Mobiltelefon übermittelt. Das Generieren von Steuersignalen basierend auf den ersten Sensorsignalen wird weiterhin ermöglicht. Bei ausreichender Zuverlässigkeit des bildbasierten Sensors wird wiederum ein Sicherheitsmodus eingeleitet, denn die Steuereinheit nimmt einen Fehler der Trägheitsnavigationseinheit an. Der Sicherheitsmodus leitet in diesem Fall ein unverzügliches Anhalten ein. Durch die fortwährende Analyse der Sensoren wird ein sicherer Betrieb des Fahrzeugs ermöglicht und es werden unverzüglich Maßnahmen zur Minimierung von Schadensfolgen bei Sensorfehlern eingeleitet.

In einer weiteren Ausführungsform wird das Erfüllen der ersten Verlässlichkeitskriterien durch Implementierung von maschinellem Lernen, insbesondere durch Klassifikation, vorzugsweise durch eine Support-Vektor-Maschine (SVM), oder durch Implementierung eines neuronalen Netzes, insbesondere eines faltenden neuronalen Netzes (CNN), ermittelt. Die Ausführungsform hat den Vorteil der individuellen Einstellbarkeit der bildbasierten Gesichtserkennung auf den konkreten Passagier. Außerdem erreichen die vorgenannten Mittel der künstlichen Intelligenz im Allgemeinen im Bereich der bildbasierten Gesichtserkennung bessere Trefferquoten und sind zuverlässiger als herkömmliche Klassifikationsmethoden, beispielsweise Entscheidungsbäume.

Für diese Ansätze der künstlichen Intelligenz ist es notwendig, im Voraus beschriftete Bilddaten, insbesondere Bilddaten mit zugeordneten Personengesichtsausdrücken, für die Klassifikation der ausgedrückten Stimmung des Passagiers bereitzustellen. Bei diesen beschrifteten Bilddaten kann es sich um einen Bilddatensatz willkürlich ausgewählter Personen, um einen individuellen Bilddatensatz des Passagiers oder einen gemischten Bilddatensatz handeln.

In einer weiteren Ausführungsform wird ein dritter Sensor, insbesondere Vitalitätsdatensensor, an einem Körperteil oder zwischen zwei Körperteilen angeordnet, um Vitalitätsparameter, insbesondere Herzfrequenzen, des Passagiers zu erfassen und dritte Sensorsignale auszugeben. Die Steuereinheit empfängt die dritten Sensorsignale, wobei bei Nichteinhalten eines Vitalitätsparameter-Wertebereichs durch die dritten Sensorsignale die zweiten Verlässlichkeitskriterien nicht erfüllt sind. In der Folge wird der Sicherheitsmodus eingenommen.

Da Vitalitätsparameter an jedem Körperteil des menschlichen Körpers erfasst werden können, kann es sich bei dem Körperteil an dem der Vitalitätsdatensensor angebracht ist um jeden Körperteil handeln. Insbesondere ist eine Anordnung am Hals, im Ohr, um das Handgelenk, um die Brust oder um den Bauch zu bevorzugen.

Auch kann der Vitalitätsdatensensor ein zweigeteilter Sensor, beispielsweise ein Brustgurt und eine Übermittlungseinheit am Handgelenk sein.

Vitalitätsdaten sind zudem nicht auf Herzfrequenzen beschränkt. Es kann auch eine kontinuierliche Überwachung des Blutdrucks, des körpereigenen Sauerstoffgehalts im Blut, des Blutbilds, des Blutzuckers, des Speichels oder anderer köpereigener, direkt oder indirekt messbarer Parameter durch den Vitalitätsdatensensor ermöglicht werden.

In einer weiteren Ausführungsform ist anstelle der Trägheitsnavigationseinheit der erste Sensor eine Augenmerkmalerfassungseinheit. Diese wird an oder in dem Wearable angebracht und ausgebildet zum Erfassen mindestens eines Auges des Passagiers und/oder dessen Merkmale, insbesondere der Lage und/oder Position und/oder Rotation und/oder Translation. Die Augenmerkmalerfassungseinheit kann erste Sensorsignale ausgeben, wobei die Steuereinheit die ersten Sensorsignale empfängt und unter deren Verwendung die ersten Steuersignale zur Steuerung des Fahrzeugs ermittelt. Bei der Augenmerkmalerfassungseinheit kann es sich um eine RGB-bildbasierte Augenmerkmalerfassungseinheit oder um eine infrarotlichtbasierte Augenmerkmalerfassungseinheit handeln.

Durch Genauigkeits- und/oder Zuverlässigkeitsanalyse der ersten Sensorsignale der Augenmerkmalerfassungseinheit und der zweiten Sensorsignale des bildbasierten Sensors, kann überprüft werden, ob die zweiten Verlässlichkeitskriterien erfüllt sind. Insbesondere muss die Steuereinheit feststellen, ob Abweichungen zwischen den die Augenmerkmale erfassenden ersten und zweiten Sensorsignalen feststellbar sind.

Eine Abweichung eines oder mehrerer der für die Erstellung erster Steuersignale relevanten Merkmale Lage und/oder Position und/oder Rotation und/oder Translation mindestens eines Auges des Passagiers muss innerhalb der zulässigen Abweichungen bzw. Toleranzen der zweiten Verlässlichkeitskriterien liegen.

Sollten eine oder mehrere der Toleranzen überschritten werden, wird auf die Selbstdiagnosedaten des bildbasierten Sensors zurückgegriffen und bei schlechter Zuverlässigkeit, beispielsweise aufgrund von schlechter Bildqualität, ein Signal über die schlechte Zuverlässigkeit dem Passagier auf das Wearable oder auf sein Mobiltelefon übermittelt. Das Generieren von ersten Steuersignalen basierend auf den ersten Sensorsignalen wird weiterhin ermöglicht.

Bei nicht eingehaltenen Toleranzen trotz ausreichender Zuverlässigkeit des bildbasierten Sensors wird ein Sicherheitsmodus eingeleitet, da die Steuereinheit Fehler in der Augenmerkmalerfassungseinheit annimmt. Der Sicherheitsmodus wird eingeleitet und führt zumindest zum Anhalten des Fahrzeugs.

Durch die fortwährende Analyse der Sensoren wird ein sicherer Betrieb des Fahrzeugs ermöglicht und es werden unverzüglich Maßnahmen zur Minimierung von Schadensfolgen bei Sensorfehlern eingeleitet. Die Sicherheit wird weiterhin durch den dritten Sensor, den Vitalitätsdatensensor, erhöht. Denn sofern die dritten Sensorsignale einen Vitalitätsparameter-Wertebereich nicht erfüllen, werden die zweiten Verlässlichkeitskriterien nicht erfüllt.

In einer Ausführungsform ist der erste Sensor ein Spracheingabesensor, der an einem Kopf-Haltemechanismus, an einem Wearable oder am Fahrzeug angebracht ist, um dem Passagier Spracheingaben zu ermöglichen. Der Spracheingabesensor ist ausgebildet erste Sensorsignale auszugeben, wobei die Steuereinheit die ersten Sensorsignale empfängt und unter deren Verwendung die ersten Steuersignale zur Steuerung des Fahrzeugs ermittelt.

Die Steuereinheit ist dazu ausgebildet, bei der Verarbeitung der ersten Sensorsignale zum Ermitteln der ersten Steuersignale eine Implementierung von neuronalen Netzen, vorzugsweise rekurrierenden neuronalen Netzen (RNN), beispielsweise eine LSTM oder eine GRU, oder faltende neuronale Netze (CNN) zu verwenden. Insbesondere für Passagiere mit eingeschränkten Sprachfähigkeiten erlauben die selbstlernenden neuronalen Netze eine Anpassung der Spracherkennung an die Stimme des Passagiers. Die durch Spracheingabe abgegebenen Steuerbefehle können einerseits in ersten Steuersignalen zum (An-) Fahren oder Stoppen des Fahrzeugs führen. Andererseits können Spracheingaben zum Bedienen einer Menüführung verwendet werden, welche dem Passagier von dem Wearable durch Projektion eines Head-up-Bildschirm angezeigt werden kann. Dadurch können weitere Befehle, beispielsweise zum Öffnen von Türen, Bestellen von Aufzügen, Drücken von Knöpfen, Bedienen von Schaltern oder weitere, externe Anwendungen abgegeben werden.

Die Steuereinheit ist ferner ausgebildet, neben den ersten Sensorsignalen des Spracheingabesensors unter Verwendung auch zweiter Sensorsignale eines zweiten Sensors, bevorzugt eines bildbasierten Sensors, die ersten Steuersignale zu ermitteln. Dabei ist der zweite Sensor auf den Kopf des Passagiers gerichtet und ausgebildet, Merkmale der Lage und/oder Position und/oder Rotation und/oder Translation eines oder beider Augen zu erfassen und die zweiten Sensorsignale auszugeben.

Als dritter Sensor wird der Vitalitätsdatensensor an einem Körperteil oder zwischen zwei Körperteilen angeordnet, um Vitalitätsparameter, insbesondere Herzfrequenzen, des Passagiers zu erfassen und dritte Sensorsignale auszugeben. Die Steuereinheit empfängt und verarbeitet die dritten Sensorsignale, wobei bei Nichteinhalten eines Vitalitätsparameter-Wertebereichs durch die dritten Sensorsignale die zweiten Verlässlichkeitskriterien nicht erfüllt werden. Der Sicherheitsmodus wird eingenommen.

Als vierter Sensor wird eine Augenmerkmalerfassungseinheit, angebracht an oder in dem Wearable, verwendet. Diese ist wie der zweite Sensor ausgebildet, mindestens ein Auge des Passagiers und/oder dessen Merkmale, insbesondere dessen Lage und/oder Position und/oder Rotation und/oder Translation zu erfassen und vierte Sensorsignale auszugeben, wobei die Steuereinheit die vierten Steuersignale empfängt und unter deren Verwendung die ersten Steuersignale zur Steuerung des Fahrzeugs ermittelt. Bei der Augenmerkmalerfassungseinheit kann es sich um eine RGB-bildbasierte Augenmerkmalerfassungseinheit oder um eine infrarotlichtbasierte Augenmerkmalerfassungseinheit handeln. Folglich werden in dieser Ausführungsform die ersten, zweiten und vierten Sensorsignale für die Ermittlung der ersten Steuersignale verwendet.

Dadurch ergibt sich ein redundantes System, wobei die Verwendung einer infrarotlichtbasierten Augenmerkmalerfassungseinheit zu einer diversitären Redundanz bei der Augenmerkmalerfassung durch den zweiten und vierten Sensor führt. Der Sicherheitsmodus wird in dieser Ausführungsform eingenommen, wenn die ersten oder die zweiten Verlässlichkeitskriterien nicht erfüllt werden.

Die Steuereinheit kann anhand der zweiten Sensorsignale Basisdaten ermitteln. Diese Basisdaten können geometrische Abmessungen und/oder Größenverhältnisse und/oder Merkmale des Gesichts und/oder Gesten sein, wobei unter Verwendung der Basisdaten die ersten Verlässlichkeitskriterien ermittelt werden.

Diese geometrisch bzw. mathematisch ausgedrückten Eigenschaften der Basisdaten charakterisieren die von Menschen wahrnehmbaren Gesichtsausdrücke und erlauben Rückschlüsse auf die vom Passagier empfundene Gefühlslage während des Betriebs des Fahrzeugs. Einerseits sollen die ersten Verlässlichkeitskriterien bei hoher Wahrscheinlichkeit eines Glückszustands, eines Entspannungszustands oder eines ähnlichen, mit positiver Stimmung assoziierten Zustands des Passagiers ein Erfüllen der ersten Verlässlichkeitskriterien bedeuten. Andererseits sollen die ersten Verlässlichkeitskriterien bei hoher Wahrscheinlichkeit von Besorgnis, eines Überraschungszustands, eines Angstzustands oder eines ähnlichen, mit negativer Stimmung assoziierten Zustands des Passagiers ein Nichterfüllen der ersten Verlässlichkeitskriterien bedeuten.

Es ist zudem möglich, dass bei Erkennung positiver Stimmung eine Rückmeldung innerhalb der Steuereinheit stattfindet und diese einen Aufnahmemodus startet. Dabei werden zumindest die von der zweiten, bildbasierten Sensor erfassten Kopfbewegungen des Passagiers erfasst und zum Training eines Algorithmus des maschinellen Lernens verwendet, um zu einem späteren Zeitpunkt die gelernten Gesten des Passagiers wiederzuerkennen und sie als Steuerbefehle interpretieren zu können. Eine solche Geste kann beispielsweise ein schnelles Kopfschütteln sein, das den Halt des Fahrzeugs auslösen soll. Durch diese Form der Gestenerkennung wird die Nutzung des Fahrzeugs für den Passagier personalisierter.

Das Erfüllen der ersten Verlässlichkeitskriterien wird durch Implementierung von maschinellem Lernen, insbesondere durch Klassifikation, vorzugsweise durch eine Support-Vektor-Maschine (SVM), oder durch Implementierung eines neuronalen Netzes, insbesondere eines faltenden neuronalen Netzes (CNN), überprüft. Zumindest bei der Anwendung von CNNs werden zudem Wahrscheinlichkeiten für die erkannten Gesichtsausdrücke von der Steuereinheit ausgewertet, um zu bestimmen, ob die ersten Verlässlichkeitskriterien erfüllt sind.

Sowohl die Methoden des maschinellen Lernens als auch die Implementierungen neuronaler Netze basieren auf bildbasierten, beschrifteten Trainingsdaten, welche auch Bilddaten von Gesichtern bzw. Gesichtsmerkmalen umfassen. Auch können diese Trainingsdaten Aufnahmen des Gesichts des Passagiers enthalten. Dies ermöglicht die Adaptierbarkeit des Algorithmus zur Erfassung von Gesichtsausdrücken auf den konkreten Passagier.

Erfindungsgemäß können durch die bildbasierte Analyse auch Rötungen, Vergrößerungen oder Verkleinerungen der Augen, inklusive der Pupillen sowie das Öffnen oder Schließen der Augen oder des Mundes und der Lippen inklusive ableitbarer Eigenschaften wie Kurzatmigkeit, erhöhter Puls oder zunehmende, gefährliche Anstrengung der Person erfasst werden. Ein Erkennen derartiger körperliche Zustände bedeutet ebenfalls das Nichterfüllen der ersten Verlässlichkeitskriterien.

In dieser Ausführungsform basieren die zweiten Verlässlichkeitskriterien auf Genauigkeits- und/oder Zuverlässigkeitsanalyse der ersten, zweiten und vierten Sensorsignale. Insbesondere erfolgt ein Genauigkeitsvergleich der erfassten Augen- bzw. Irismerkmale des zweiten, bildbasierten Sensors mit denen des vierten Sensors, der Augenmerkmalerfassungseinheit.

Basierend auf der Genauigkeits- und/oder Zuverlässigkeitsanalyse der zweiten Sensorsignale des bildbasierten Sensors und der vierten Sensorsignale der Augenmerkmalerfassungseinheit, kann die Steuereinheit überprüfen, ob die Abweichung eines oder mehrere der Merkmale Lage und/oder Position und/oder Rotation und/oder Translation des Auges des Passagiers innerhalb einer oder mehrerer Toleranzen liegt.

Sollten eine oder mehrere der Toleranzen überschritten werden, wird auf die Selbstdiagnosedaten des bildbasierten Sensors zurückgegriffen und bei schlechter Zuverlässigkeit, beispielsweise aufgrund von schlechter Bildqualität, ein Signal über die schlechte Zuverlässigkeit dem Passagier über das Wearable oder auf sein Mobiltelefon übermittelt. Das Generieren von Steuerbefehlen basierend auf den vierten Sensorsignalen wird weiterhin ermöglicht.

Sofern bei Überschreiten einer oder mehrerer der Toleranzen keine geringe Zuverlässigkeit des zweiten, bildbasierten, Sensors festgestellt werden kann, wird der Sicherheitsmodus eingenommen und das Fahrzeug zumindest angehalten. Der Grund dafür ist, dass bei ausreichender Zuverlässigkeit des zweiten Sensors die Steuereinheit annimmt, dass ein Fehler der Augenmerkmalerfassungseinheit vorliegt.

Bei Nichtverletzen der ersten und zweiten Verlässlichkeitskriterien kann das augenmerkmalbasierte Steuern des Fahrzeugs durch Einnehmen eines Initialzustands, beispielsweise das Fixieren eines Punktes in der Mitte des Augenpaares, gestartet werden. Auch kann eine Abfolge von Gesten, beispielsweise ein dreimaliges Augenblinzeln oder bestimmte vordefinierte Augenbewegungsmuster, das Starten einer augenmerkmalbasierten Steuerung bedeuten. Auch kann der erste Spracheingabesensor zur Erfassung eines Startbefehls verwendet werden.

Die Steuerung erfolgt anschließend durch Zuordnung einer Blickrichtung zur gewünschten Bewegungsrichtung. Das Fahrzeug kann angehalten werden, indem eine vordefinierte Geste, beispielsweise das Schließen der Augen, oder ein Augenblinzeln, vom zweiten Sensor oder dem vierten Sensor erfasst wird. Auch kann das Fahrzeug durch Spracheingabe unter Verwendung des ersten Sensors, des Spracheingabesensors, zum Anhalten gebracht werden.

Durch die fortwährende Analyse der Sensoren wird ein sicherer Betrieb des Fahrzeugs ermöglicht und es werden unverzüglich Maßnahmen zur Minimierung von Schadensfolgen bei Sensorfehlern eingeleitet. Die Sicherheit wird zudem durch die Verwendung des dritten Sensors, des Vitalitätsdatensensors, erhöht, denn sofern die dritten Sensorsignale einen Vitalitätsparameter-Wertebereich nicht erfüllen, werden die zweiten Verlässlichkeitskriterien nicht erfüllt. Der Sicherheitsmodus wird eingenommen.

In einer Ausführungsform wird anstelle des vierten Sensors, der Augenmerkmalerfassungseinheit, ein fünfter Sensor, eine Gehirn-Steuereinheit-Schnittstelle bzw. ein Eingabegerät auf und/oder im Kopf des Passagiers angeordnet. Diese ist ausgebildet, fünfte Sensorsignale auszugeben, wobei die Steuereinheit die fünften Sensorsignale empfängt und unter deren Verwendung die ersten Steuersignale zur Steuerung des Fahrzeugs ermittelt. Die Gehirn-Steuereinheit-Schnittstelle wird als primäre Datenquelle für die Generierung der ersten Steuersignale verwendet. Weiterhin werden anhand des bildbasierten Sensors zumindest die ersten Verlässlichkeitskriterien ermittelt und bei Nichterfüllen der ersten Verlässlichkeitskriterien wird der Sicherheitsmodus eingeleitet.

Vordefinierte Steuerbefehle werden in der Steuereinheit den fünften Steuersignalen zugeordnet, sodass ein von den fünften Sensorsignalen erfasster Gedanke des Passagiers einem gewünschten Steuerbefehl zugeordnet wird. Weiterhin können in den fünften Sensorsignalen erfasste, abstrakte Gedankenmuster des Passagiers einem maschinellen Lernalgorithmus in der Steuereinheit bereitgestellt werden, wobei der Lernalgorithmus aus den bereitgestellten Daten lernt und anhand erkannter Steuerbefehle des Passagiers die ersten Steuersignale ermittelt.

Weiterhin können die Signale jedoch auch genutzt werden um festzustellen, ob der Passagier in Panik geraten ist, beispielsweise, weil er sich in einem gefährlichen körperlichen Zustand befindet.

Durch die Kombination mit dem ersten Spracheingabesensor, dem zweiten, bildbasierten Sensor sowie dem dritten Sensor dieser Ausführungsform, dem Vitalitätsdatensensor, können gefährliche Situationen leichter klassifiziert werden und das Fahren mit dem Fahrzeug wird sicherer.

In einer Ausführungsform werden alle bisher verwendeten Sensoren zum Bereitstellen einer noch sichereren und zuverlässigeren Lösung kombiniert. Als erster Sensor wird die Trägheitsnavigationseinheit, angebracht an oder in dem Wearable, verwendet, um die Lage und/oder Position und/oder Rotation und/oder Translation des Kopfs des Passagiers zu erfassen und erste Sensorsignale an die Steuereinheit zu übermitteln und unter deren Verwendung die ersten Steuersignale zur Steuerung des Fahrzeugs zu ermitteln. Der zweite, bildbasierte, Sensor kann den Kopf des Passagiers erfassen und zweite Sensorsignale der Steuereinheit übermitteln, wobei unter deren Verwendung die ersten Verlässlichkeitskriterien bestimmt werden sowie die ersten Steuersignale zur Steuerung des Fahrzeugs ermittelt werden.

Ein dritter Sensor, der Vitalitätsdatensensor, übermittelt dritte Sensorsignale an die Steuereinheit. Diese bilden Vitalitätsparameter, insbesondere Herzfrequenzen, des Passagiers ab, wobei bei Nichteinhalten eines Vitalitätsparameter-Wertebereichs durch die dritten Sensorsignale die zweiten Verlässlichkeitskriterien nicht erfüllt werden.

Als vierter Sensor wird eine Augenmerkmalerfassungseinheit verwendet, angebracht an oder in dem Wearable, und dazu ausgebildet, eines oder beide Augen des Passagiers, insbesondere deren Lage und/oder Position und/oder Rotation und/oder Translation, des Passagiers zu erfassen und vierte Sensorsignale auszugeben, wobei die Steuereinheit die vierten Sensorsignale empfängt und unter deren Verwendung die ersten Steuersignale zur Steuerung des Fahrzeugs ermittelt.

Basierend auf der Genauigkeits- und/oder Zuverlässigkeitsanalyse der ersten Sensorsignale der Trägheitsnavigationseinheit und der zweiten Sensorsignale des bildbasierten Sensors, kann die Steuereinheit überprüfen, ob die Abweichung eines oder mehrerer der Merkmale Lage und/oder Position und/oder Rotation und/oder Translation des Kopfs des Passagiers innerhalb einer oder mehrerer Toleranzen liegen.

Basierend auf der Genauigkeits- und/oder Zuverlässigkeitsanalyse der zweiten Sensorsignale des bildbasierten Sensors und der vierten Sensorsignale der Augenmerkmalerfassungseinheit, kann die Steuereinheit überprüfen, ob die Abweichung eines oder mehrerer der Merkmale Lage und/oder Position und/oder Rotation und/oder Translation des Auges oder der Augen des Passagiers innerhalb einer oder mehrerer Toleranzen liegt.

Der fünfte Sensor, insbesondere die Gehirn-Steuereinheit-Schnittstelle bzw. das Eingabegerät, ist auf und/oder im Kopf des Passagiers angeordnet und ausgebildet fünfte Sensorsignale auszugeben, wobei die Steuereinheit die fünften Sensorsignale empfängt und unter deren Verwendung die ersten Steuersignale zur Steuerung des Fahrzeugs ermittelt. Die fünften Sensorsignale können Steuerbefehle des Passagiers zum Starten und Stoppen umfassen.

Als sechster Sensor wird der Spracheingabesensor verwendet, der insbesondere ein Kopf-Haltemechanismus-Spracheingabesensor, ein Wearable-Spracheingabesensor oder ein Fahrzeug-Spracheingabesensor sein kann und ausgebildet sein kann sechste Sensorsignale auszugeben, wobei die Steuereinheit die sechsten Sensorsignale empfängt und unter deren Verwendung die ersten Steuersignale zur Steuerung des Fahrzeugs ermittelt. Durch den Spracheingabesensor können Passagierbefehle zum Starten oder Stoppen des Fahrzeugs durch die sechsten Sensorsignale an die Steuereinheit übermittelt werden.

Sofern eine oder mehrere der Toleranzen nicht erfüllt werden, sind die zweiten Verlässlichkeitskriterien nicht erfüllt. Sollten die ersten oder zweiten Verlässlichkeitskriterien nicht erfüllt sein, nimmt die Vorrichtung den Sicherheitsmodus ein.

Wenn die Verlässlichkeitskriterien erfüllt sind, können erste Steuersignale zur Bewegung des Fahrzeugs von der Steuereinheit an die Fahrzeug-Aktuatorik übermittelt werden. Die vierten Sensorsignale definieren als Teil der ersten Steuersignale die Fahrtrichtung, während die ersten Sensorsignale die gewünschte Geschwindigkeit in der Fahrtrichtung definieren.

In einer weiteren Ausführungsform ist zudem eine erste umgebungserfassende Sensorbaugruppe, insbesondere eine Ultraschallbaugruppe und/oder eine LIDAR-Sensorbaugruppe und/oder eine bildbasierte Sensorbaugruppe und/oder eine RADAR-Sensorbaugruppe, am Fahrzeug zum Erfassen der Umgebung angebracht und ausgebildet, erste Sensorbaugruppensignale auszugeben, wobei die Steuereinheit erste Sensorbaugruppensignale empfängt und unter deren Verwendung die ersten Steuersignale ermittelt.

Die Umgebung kann auch von einem bildbasierten Rückseitensensor und/oder einem Wearable-Bildsensor erfasst und entsprechende Sensorsignale können an die Steuereinheit übermittelt werden. Unter deren Verwendung kann die Steuereinheit die ersten Steuersignale ermitteln.

Dabei können die erfassten Umgebungsinformationen von der Steuereinheit ausgewertet werden und es kann bei kritischer Annäherung an Hindernisse, Gefälle, Steigungen oder Ähnlichem der Sicherheitsmodus eingenommen werden.

In einer Ausführungsform ist das Wearable und/oder ein Mobiltelefon ausgebildet und angeordnet, siebte Sensorsignale, insbesondere Passagierzieleingabe-Sensorsignale, auszugeben, wobei die Steuereinheit zuvor verfügbare Zieleingabe-Auswahldaten dem Wearable und/oder dem Mobiltelefon für die Passagierzieleingabe übermittelt hat.

Die Zieleingabe-Auswahldaten entstehen durch Auswertung der Sensorsignale mindestens der ersten umgebungserfassenden Sensorbaugruppe durch die Steuereinheit. Bei den Zielen kann es sich grundsätzlich um alle Elemente handeln, deren Kontur von einer RADAR- und/oder LIDAR- und/oder Ultraschall- und/oder bildbasierten Sensorbaugruppe erfasst werden können und die sich im Sichtfeld des Passagiers befinden.

Für bildbasierte Sensordaten können trainierte, faltende neuronale Netze (CNNs) verwendet werden. Der für das Training eines oder mehrerer CNNs notwendige, bildbasierte Trainingsdatensatz umfasst eine Vielzahl verschiedener, beschrifteter Bilddaten, die in Gebäuden sowie außerhalb von Gebäuden von verschiedenen Kameras sowie bei verschiedenen Belichtungssituationen aufgenommen wurden, sodass die CNNs eine Vielzahl von durch die umgebungserfassende Sensorbaugruppe erfassten Objekte erkennen und dem Passagier als Zieleingabe-Auswahldaten bereitstellen können.

Zieleingabe-Auswahldaten werden dem Passagier, wenn das Wearable verwendet wird, nach Vorbild eines Head-up-Bildschirms in sein Sichtfeld projiziert. Mobiltelefone erlauben eine Bildschirmanzeige. Die Steuereinheit empfängt die siebten Sensorsignale und ermittelt unter deren Verwendung die ersten Steuersignale.

Dabei hat die Steuereinheit die Aufgabe eine Trajektorie zum Zielort zu berechnen, geeignete erste Steuersignale bereitzustellen sowie während der Fahrt zum Zielort basierend auf den Echtzeitdaten der umgebungserfassenden Sensoren Anpassungen der Trajektorie, beispielsweise aufgrund der sich verändernden Umgebung, vorzunehmen, und angepasste erste Steuersignale bereitzustellen.

Bei Verwendung von LIDAR- oder RADAR-Sensorbaugruppen werden dreidimensionale, umgebungsabbildende Karten erzeugt. Den dreidimensionalen Punktewolken dieser Karten können durch maschinelles Lernen Bildmerkmale aufgenommener Bilder der Umgebung zugeordnet werden. Dadurch ist es möglich, eine dreidimensionale Trajektorie zum Zielobjekt in der Umgebung zu generieren. Das Fahrzeug bewegt sich entlang dieser Trajektorie, indem die generierten ersten Steuersignale der Fahrzeug-Aktuatorik bereitgestellt werden, um die Distanz zum Zielort bzw. -objekt zu minimieren. Wenn die minimale Distanz zum Ziel erreicht ist, hält das Fahrzeug an.

Auch ist es möglich dem Passagier in Echtzeit Zieleingabe-Auswahldaten auf sein Head-up-Sichtfeld im Wearable zu projizieren, indem der Wearable-Bildsensor und/oder mindestens eine, bevorzugt bildbasierte, umgebungserfassende Sensorbaugruppe angeordnet ist sich anhand der Blickrichtung des Passagiers, auf die Umgebung auszurichten und erste Sensorbaugruppensignale der Steuereinheit zu übermitteln. Die Steuereinheit ermittelt unter Verwendung der ersten Sensorbaugruppensignale Zieleingabe-Auswahldaten und übermittelt diese an das Wearable zum Projizieren in den Head-up-Bildschirm. Bevorzugt geschieht dies über eine LTE- oder eine 5G-Verbindung.

Der Passagier kann dadurch eine Zielobjektauswahl unmittelbar anhand der Echtzeit-Bilder bzw. eines Echtzeit-Videos durchführen und es werden siebte Sensorsignale, insbesondere Passagierzieleingabe-Sensorsignale vom Wearable an die Steuereinheit übermittelt. Die Steuereinheit kann unter Verwendung der siebten Sensorsignale die ersten Steuersignale ermitteln.

Die zweiten, dritten und fünften Sensorsignale werden von der Steuereinheit verwendet, um Rückmeldung darüber zu geben, ob das Fahrzeug sich aus Passagiersicht auf dem richtigen Weg zum Ziel befindet. Dabei wird der Gesichtsausdruck des Passagiers, die Herzfrequenz sowie die Gedanken des Passagiers überwacht, wobei der Sicherheitsmodus eingenommen wird, wenn anhand der Sensorsignale ein kritischer körperlicher Zustand des Passagiers erfasst wird. Auch kann jederzeit die automatisierte Ansteuerung eines ausgewählten Zielobjekts durch manuelle Übersteuerung durch den Passagier unterbrochen oder beendet werden.

In einer Ausführungsform wird ein achter Sensor, insbesondere ein Positionssensor, beispielsweise ein GPS-Sensor oder ein Sensor zur Ortung innerhalb von Gebäuden, angeordnet und ausgebildet achte Sensorsignale auszugeben, verwendet, wobei die Steuereinheit die achten Sensorsignale empfängt und unter deren Verwendung die ersten Steuersignale ermittelt.

Der Positionssensor erlaubt die Darstellung einer Karte mit der aktuellen Position des Fahrzeugs bzw. des Passagiers mittels der Head-up-Projektion des Wearable oder auf sein Mobiltelefon. Die Verwendung einer Karte in Verbindung mit den dargestellten Zieleingabe-Auswahldaten erlaubt das Auswählen von Zielorten oder -objekten, die nicht mehr von umgebungserfassenden Sensoren wahrgenommen werden können. Die Auswahl eines Ziels kann durch Führung eines virtuellen Mauszeigers per Augensteuerung in der Head-up-Projektion des Wearable oder durch Auswahl auf dem Mobiltelefon erfolgen.

Basierend auf der Zieleingabe des Passagiers werden die siebten Passagierziel-Sensorsignale der Steuereinheit bereitgestellt. Unter Verwendung der Karte und der achten Sensorsignale des Positionssensors kann eine Trajektorie zum Zielort bzw. Objekt bestimmt werden und entsprechende, erste Steuersignale können bereitgestellt werden.

Somit hat die Steuereinheit die Aufgabe, eine Trajektorie zum Zielort zu berechnen, geeignete erste Steuersignale bereitzustellen, sowie während der Fahrt zum Zielort basierend auf den Echtzeitdaten der umgebungserfassenden Sensoren, Anpassungen der Trajektorie, beispielsweise aufgrund der sich verändernden Umgebung, vorzunehmen, und angepasste erste Steuersignale bereitzustellen. Nach Erreichen dies Zielorts bzw. Zielobjekts hält das Fahrzeug an.

Die zweiten, dritten und fünften Sensorsignale können von der Steuereinheit verwendet werden, um Rückmeldung darüber zu geben, ob das Fahrzeug sich aus Passagiersicht auf dem richtigen Weg zum Ziel befindet. Dabei wird der Gesichtsausdruck des Passagiers, die Herzfrequenz sowie die Gedanken des Passagiers überwacht, wobei der Sicherheitsmodus eingenommen wird, wenn anhand der Sensorsignale ein kritischer körperlicher Zustand des Passagiers erfasst wird.

Auch kann jederzeit die automatisierte Ansteuerung eines ausgewählten Zielobjekts durch manuelle Übersteuerung durch den Passagier unterbrochen oder beendet werden.

In einer Ausführungsform umfasst die Vorrichtung eine Fernkommunikationsvorrichtung, beispielsweise eine LTE- oder 5G-Kommunikationsvorrichtung oder eine Mobiltelefonie-Kommunikationsvorrichtung, die am Fahrzeug angeordnet und ausgebildet ist, einen Signalaustausch mit der Steuereinheit und externen Mobilfunkanbietervorrichtungen durchzuführen. Dabei ist das Wearable ebenfalls über eine LTE- oder 5G-Verbindung mit der Steuereinheit verbunden, sodass eine (Video-)Kommunikation durchgeführt werden kann oder Bilder oder Videos, die von dem Wearable-Bildsensor aufgenommen wurden, in persönlichen Nachrichten oder über soziale Netzwerke Anderen bereitgestellt werden.

Des Weiteren kann im Fall eines eingeleiteten Sicherheitsmodus, beispielsweise aufgrund eines erkannten kritischen körperlichen Zustands des Passagiers, mindestens ein Notfallkontakt automatisch kontaktiert werden.

In einer Ausführungsform umfasst die Vorrichtung zudem Fahrdynamik-Sensoren, insbesondere Raddrehzahlsensoren und/oder Trägheitsnavigationseinheiten mit integrierten Beschleunigungsmessern und/oder Drehratenmessern und/oder Kompass, angeordnet am Fahrzeug und ausgebildet, neunte Sensorsignale auszugeben, wobei die Steuereinheit die neunten Sensorsignale empfängt und unter deren Verwendung die ersten Steuersignale ermittelt. Diese Sensorik erlaubt insbesondere die Regelung der Fahrdynamik durch Rückführung tatsächlicher Fahrdynamik-Sensorgrößen an die Steuereinheit, um gegenüber einer Steuerung eine genauere Umsetzung der gewünschten Steuerungsbefehle zu ermöglichen.

In einer weiteren Ausführungsform wird der bildbasierte Frontsensor für die Erfassung des Gesichts des Passagiers durch einen bildbasierten Fahrzeug-Sensor ersetzt, wobei dieser bildbasierte Sensorsignale der Steuereinheit übermittelt und unter deren Verwendung die Steuereinheit die ersten Steuersignale ermittelt sowie das Erfüllen der ersten Verlässlichkeitskriterien bewertet wird.

Weitere vorteilhafte Ausführungsformen ergeben sich aus den Unteransprüchen.

Nachfolgend wird die Erfindung auch hinsichtlich weiterer Merkmale und Vorteile anhand von Ausführungsbeispielen beschrieben, die anhand der Abbildungen näher erläutert werden.

Hierbei zeigen:
- Fig. 1: ein Ablaufdiagramm der Vorrichtung nach einem Ausführungsbeispiel;
- Fig. 2: ein Fahrzeug mit Wearable, Trägheitsnavigationseinheit, bildbasiertem Sensor, Steuereinheit und Fahrzeug-Aktautoren;
- Fig. 3: das Fahrzeug gemäß Fig. 2 mit Vitalitätsdatensensor;
- Fig. 4: das Fahrzeug gemäß Fig. 3 ohne Trägheitsnavigationseinheit, mit Augenmerkmalerfassungseinheit;
- Fig. 5: das Fahrzeug gemäß Fig. 4, mit Wearable-Spracheingabesensor;
- Fig. 6: das Fahrzeug gemäß Fig. 3, ohne Wearable, ohne Trägheitsnavigationseinheit, mit Gehirn-Steuereinheit-Schnittstelle bzw. -Eingabegerät, ohne Wearable-Spracheingabesensor, mit Fahrzeug-Spracheingabesensor;
- Fig. 7: das Fahrzeug gemäß Fig. 6, mit Wearable, mit Trägheitsnavigationseinheit, mit Augenmerkmalerfassungseinheit, mit Spracheingabesensor;
- Fig. 8: das Fahrzeug gemäß Fig. 7, mit Wearable-Bildsensor, mit umgebungserfassender Sensorbaugruppe; und
- Fig. 9: das Fahrzeug gemäß Fig. 8 mit Positionssensor, mit Fernkommunikationsvorrichtung, mit Wearable-Spracheingabesensor, ohne Fahrzeug-Spracheingabesensor.

In der nachfolgenden Beschreibung und in den Zeichnungen werden für gleiche und gleichwirkende Teile dieselben Bezugszeichen verwendet.

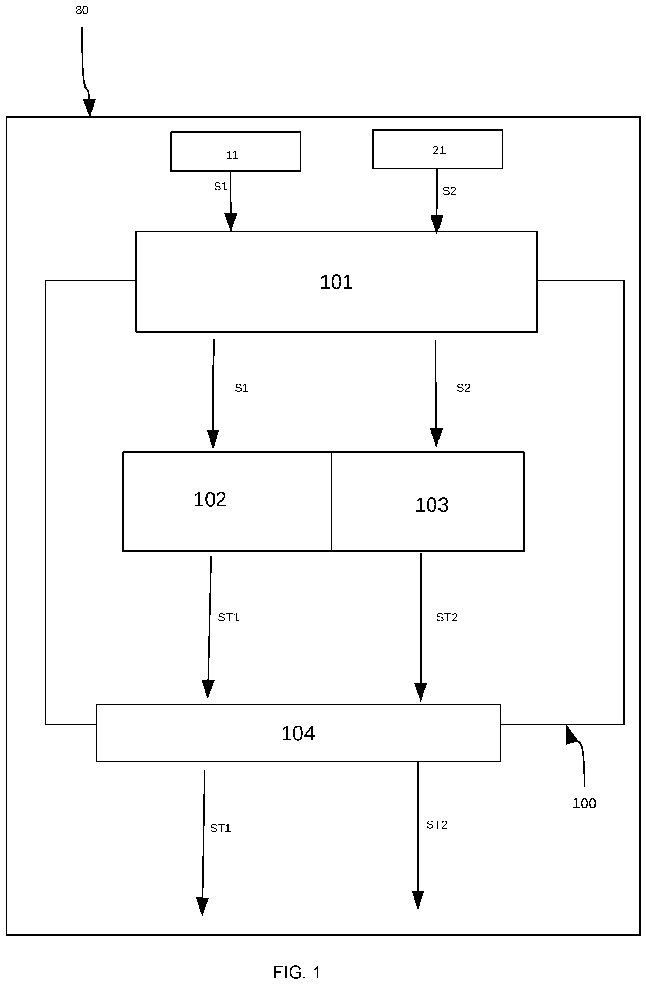

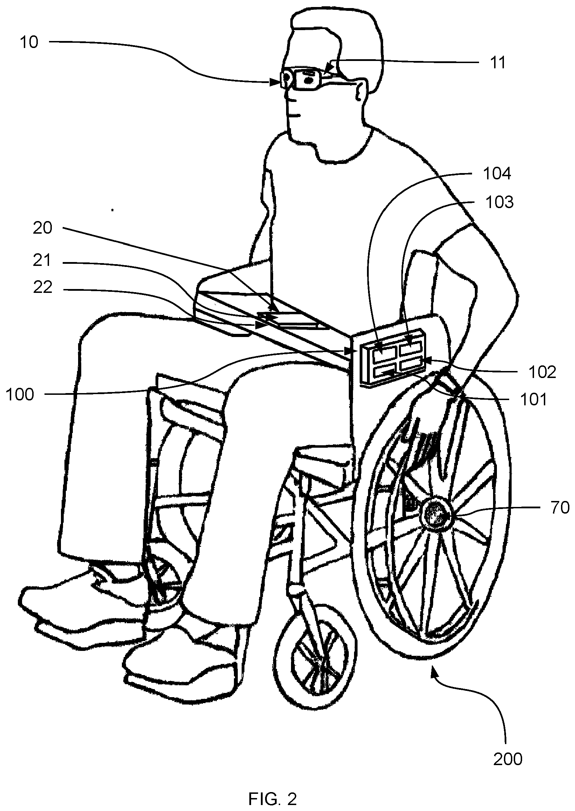

Fig. 1 zeigt ein Ablaufdiagramm einer Vorrichtung 80 zum Navigieren eines Rollstuhls 200 (vgl. Fig. 2).

Die Vorrichtung 80 umfasst in einem Ausführungsbeispiel zwei Sensoren. Als erster Sensor wird eine Trägheitsnavigationseinheit 11 verwendet, die an einem tragbaren Computersystem, Wearable 10, am Kopf eines Rollstuhlfahrers eines Rollstuhls 200 (z. B. Fig. 2) angebracht ist. Beispielsweise ist sie Bestandteil von Google Glass, die von dem Rollstuhlfahrer getragen wird. Die Trägheitsnavigationseinheit 11 erfasst Lage und Rotation des Kopfs des Rollstuhlfahrers und gibt diese Daten als erste Sensorsignale S1 aus. Als zweiter Sensor dient ein bildbasierter Frontsensor 21 eines Mobiltelefons 20 (Fig. 2), wobei das Mobiltelefon 20 so an einem Bauteil des Rollstuhls 200 fixiert ist, dass der Frontsensor 21 den Kopf des Rollstuhlfahrers erfasst. Dabei generierte Bilddaten des Fronsensors 21 werden vom Mobiltelefon 20 als zweite Sensorsignale S2 ausgegeben.

Eine Steuereinheit 100 ist kommunikativ, z. B. über Bluetooth, mit Google Glass verbunden und empfängt die ersten Sensorsignale S1. Die zweiten Sensorsignale S2 können über eine USB Verbindung an die Steuereinheit 100, insbesondere an eine Sensorsignal-Empfangseinheit 101, übertragen werden. In einer Steuersignal-Ermittlungseinheit 102 der Steuereinheit 100 werden basierend auf den ersten Sensorsignalen S1 erste Steuersignale ST1 zur Steuerung des Rollstuhls 200 erstellt. Basierend auf den zweiten Sensorsignalen S2 ermittelt eine Verlässlichkeitskriterien-Prüfeinheit 103, ob die ersten Steuersignale ST1 erste Verlässlichkeitskriterien erfüllen.

Ein Sicherheitsmodus wird eingenommen, wenn die Verlässlichkeitskriterien-Prüfeinheit 103 bestimmt, dass die ersten Steuersignale ST1 nicht die ersten Verlässlichkeitskriterien erfüllen.

Die Verlässlichkeitskriterien-Prüfeinheit 103 ermittelt anhand der zweiten Sensorsignale S2 Basisdaten. Die Basisdaten umfassen geometrische Abmessungen, Größenverhältnisse und Merkmale des Gesichts. Diese geometrisch bzw. mathematisch ausgedrückten Eigenschaften der Basisdaten charakterisieren die wahrnehmbaren Gesichtsausdrücke einer Person und erlauben dadurch die Klassifikation der vom Rollstuhlfahrer empfundenen Gefühlslage bzw. Emotionen während des Betriebs des Rollstuhls 200.

Vorrichtungen zur Klassifikation von Emotionen einer Person anhand erkannter geometrischer Abmessungen, Größenverhältnisse und Merkmale des Gesichts sind bekannt. So beschreibt beispielsweise die IN 00554CH2014 A ein Verfahren und eine Vorrichtung mit denen eine gegenüber zuvor bestimmten, neutralen Gesichtsausdrücken veränderte Mimik von Personen erkannt und einer oder mehreren Emotionen zugeordnet werden kann.

Die Lehre der IN 00554CH2014 A nutzt zum Erkennen von Gesichtern, um sodann Abmessungen, Größenverhältnisse und Formen von Merkmalen wie Augen, Nase, Mund oder Kinn zu bestimmen, sogenannte beschränkte, lokale Modellmethoden (Constrained Local Model (CLM) method). Weiterhin kann eine Support-Vektor-Maschine (SVM) anhand einer Vielzahl zuvor beschrifteter Bilddaten von Gesichtern darauf trainiert werden, aus den zuvor erkannten geometrischen Gesichtsmerkmalen Aktionen wie das Schließen eines Auges oder das Öffnen eines Mundes abzuleiten. Abschließend wird durch ein statistisches Verfahren (Discriminative Power Concept) die Wahrscheinlichkeit für eine bestimmte Emotion einer Person bestimmt, indem bewertet wird, wie wahrscheinlich eine Aktion ist, sofern eine bestimmte Emotion vorliegt, abzüglich der Wahrscheinlichkeit dieser Aktion, wenn diese Emotion nicht vorliegt.

Konkret kann das bedeuten, dass bei Erkennen von nach oben gezogenen Mundwinkeln gegenüber einer bekannten, neutralen Mundposition ein Glückszustand des Rollstuhlfahrers ermittelt wird, da keine der bekannten, weiteren Emotionen durch nach oben gezogene Mundwinkel charakterisiert ist. Die IN 00554CH2014 A nennt die Emotionen Wut, Angst, Glückszustand, Überraschungszustand, Ekel, Traurigkeit als erkennbar. Zudem werden jeweils Wahrscheinlichkeiten der einzelnen Emotionen ausgegeben.

Das Ausführungsbeispiel bietet die Möglichkeit und damit den Vorteil der individuellen Einstellbarkeit der bildbasierten Gesichtsmerkmalserkennung auf den konkreten Rollstuhlfahrer, sofern die Support-Vektor-Maschine SVM (auch) mit beschrifteten Bilddaten von Gesichtsmuskelbewegungen des Rollstuhlfahrers trainiert wird. Zudem können die der Klassifikation der Emotion zugrundeliegenden, erfassten Merkmale des Gesichts in Echtzeit evaluiert werden. Dazu werden diese der Bilddatenfolge (Videosequenz) der zweiten Sensorsignale S2 des bildbasierten Frontsensors 21 überlagert.

Alternativ ist nach einem weiteren Ausführungsbeispiel gemäß Fig. 1 die Grundlage der Klassifikation der Emotionen des Rollstuhlfahrers eine Implementierung eines faltenden neuronalen Netzes (CNN), welches in der Verlässlichkeitskriterien-Prüfeinheit 103 auf die zweiten Sensorsignale S2 angewandt wird. Für diesen Ansatz der künstlichen Intelligenz ist es notwendig, im Voraus beschriftete Bilddaten, also Bilddaten mit zugeordneten Emotionen einer Person, für die Klassifikation der ausgedrückten Emotion des Rollstuhlfahrers bereitzustellen. Die geometrischen Merkmale des Gesichts, deren Abmessungen und Größenverhältnisse werden dabei aufgrund der abstrakten Darstellung der trainierten Gewichte des neuronalen Netzes während des Trainings nicht sichtbar.

Die Trefferquote und damit die Qualität der Emotionserkennung ist anhand eines Testdatensatzes zu beurteilen. Das faltende neuronale Netz ist derart ausgebildet, dass der Anzahl zu erkennender Emotionen des Rollstuhlfahrers je ein Wahrscheinlichkeitswert zugeordnet ist. Die Nutzung des neuronalen Netzes hat den Vorteil, dass ähnlich der menschlichen Wahrnehmung von Emotionen in Gesichtern, nicht ein oder wenige Merkmale des Gesichts Berücksichtigung finden, sondern die Emotion des Rollstuhlfahrers im Zusammenspiel aller durch die zweiten Sensorsignale S2 übermittelten Bilddaten ermittelt wird. So können durch die CNN-Trainingsdaten auch personenspezifische Merkmale des Gesichts, wie beispielsweise Lachfalten, implizit für die Emotionserkennung berücksichtigt werden, ohne dass diese explizit in den Trainingsdaten charakterisiert sind.

Unabhängig von der Wahl des Mittels zur Klassifikation der Emotionen, SVM oder CNN, sollen die ersten Verlässlichkeitskriterien bei hoher Wahrscheinlichkeit eines Glückszustands, eines Entspannungszustands oder eines ähnlichen, mit positiver Emotion assoziierten Zustands des Rollstuhlfahrers ein Erfüllen der ersten Verlässlichkeitskriterien bedeuten.

Eine erkannte, positive Stimmung bedeutet jedoch nicht nur das Erfüllen der ersten Verlässlichkeitskriterien. Zudem erfolgt in der Steuersignal-Ermittlungseinheit 102 auch eine Zeitreihenaufzeichnung der Lage- und Rotationsdaten des Kopfs des Rollstuhlfahrers, welche einem Algorithmus des maschinellen Lernens zur Verfügung gestellt werden. Dieser kann die von den Daten abgebildeten, individuellen Bewegungsabfolgen des Kopfs des Rollstuhlfahrers erfassen und verarbeiten, wodurch die Rollstuhlsteuerung den persönlichen Anforderungen angepasst wird.

Es ist möglich, für jeden Rollstuhlfahrer die maximal auftretenden Neigungen des Kopfs in die Richtungen vorne/hinten bzw. links/rechts beim Betrieb des Rollstuhls 200 sowie die dazugehörigen Rotationsgeschwindigkeiten zu bestimmen. So kann für einen Rollstuhlfahrer mit noch vorhandener, aber stark eingeschränkter Bewegungsfreiheit des Genicks bereits eine geringe Neigung des Kopfs in eine Richtung eine maximale Bewegung des Rollstuhls in die gewünschte Richtung bedeuten. Durch das zusätzliche Aufzeichnen der Rotationsgeschwindigkeiten über die Lagewinkel des Kopfs ist es zudem möglich, bei krankheitsbedingt zitternden oder bei nervösen Rollstuhlfahrern unvermeidlich resultierende Kopfneigungen nicht als Steuerbefehle zu interpretieren. Auch kann dadurch für Rollstuhlfahrer mit ungewollt bzw. unkontrolliert ruckartigen Kopfbewegungen ein individuelles, sanfteres Fahrverhalten des Rollstuhls ermöglicht werden.

Als Algorithmen des maschinellen Lernens bieten sich für diese Aufgabe Regressionsalgorithmen an. Dadurch muss eine vom Rollstuhlfahrer gewünschte Charakteristik der Steuerung des Rollstuhls 200 nicht nur auf einer einzigen, aufgezeichneten Lage-Zeit- oder Rotationsgeschwindigkeit-Lage-Funktion beruhen. Vielmehr können mehrere, zeitlich beabstandet aufgezeichnete Funktionen zum Abbilden einer gemittelten, individuellen Charakteristik der Steuerung des Rollstuhls 200 verwendet werden.

Bei Erfüllen der ersten Verlässlichkeitskriterien erfolgt die Steuerung, indem ein Nach-oben-Neigen des Kopfs je nach Voreinstellung ein Vorwärts- oder Rückwärtsfahren des Rollstuhls 200 bedeutet. Ein Neigen des Kopfs nach links bzw. rechts führt zu einer Fahrt des Rollstuhls nach links bzw. rechts. Zum Anhalten des Rollstuhls wird der Kopf in eine zuvor festgelegte Normalposition bzw. Neutralposition gebracht.

Die ersten Verlässlichkeitskriterien sollen bei hoher Wahrscheinlichkeit einer Unfähigkeit zum Steuern des Rollstuhls 200 wie Besorgnis, einem Überraschungszustand, einem Angstzustand, einem Unzufriedenheitszustand oder einem ähnlichen, mit negativer Emotion assoziierten Zustand des Rollstuhlfahrers, ein Nichterfüllen der ersten Verlässlichkeitskriterien bedeuten. Beispielsweise signalisieren nach unten gezogene Mundwinkel einen Unzufriedenheitszustand. Überraschungs- und Angstzustände können durch das Erkennen von weit aufgerissenen Augen oder Mund identifiziert werden. Eine Unfähigkeit zum Steuern des Rollstuhls 200 wird aus dem Schließen der Augen für einen Zeitraum, der länger ist als ein Zeitraum zum Blinzeln, abgeleitet. Die dem faltenden neuronalen Netz (CNN) bereitgestellten Trainingsdaten berücksichtigen diese Zuordnung von Gesichtsausdrücken zu Emotionen bzw. Zuständen. Gleiches gilt für das Ausführungsbeispiel zur Klassifikation der Emotionen durch die Support-Vektor Maschine (SVM).

Der Sicherheitsmodus ist ein eingeschränkter Betriebsbereich der Vorrichtung 80, wobei im Sicherheitsmodus zweite Steuersignale ST2 von der Verlässlichkeitskriterien-Prüfeinheit 103 ausgegeben werden. Dabei werden bei Unfähigkeit zum Steuern des Rollstuhls 200, beispielsweise wenn der Rollstuhlfahrer die Augen geschlossen hat, die zweiten Steuersignale ST2 ein Abbremsen und Anhalten des Rollstuhls bewirken.

Bei Erhöhung der Wahrscheinlichkeit für einen Überraschungszustand, einen Angstzustand oder einen Besorgniszustand des Rollstuhlfahrers verwendet die Verlässlichkeitskriterien-Prüfeinheit 103 die ersten Sensorsignale S1 der Trägheitsnavigationseinheit 11 für die Bestimmung der zweiten Steuersignale ST2. Wenn die ermittelte Lage oder Rotation des Kopfs des Rollstuhlfahrers in einem unzulässigen Wertebereich liegt, bewirken die zweiten Steuersignale ST2 ein Anhalten des Rollstuhls 200. Wenn die Lage und Rotation des Kopfs des Rollstuhlfahrers hingegen in einem zulässigen Wertebereich liegt, wird lediglich die maximale Geschwindigkeit des Rollstuhls 200 reduziert, da die Verlässlichkeitskriterien-Prüfeinheit 103 annimmt, dass diese bislang für den Rollstuhlfahrer unangemessen hoch war.

Die zweiten Steuersignale ST2 sind ungleich den ersten Steuersignalen ST1, können jedoch beide von der Vorrichtung 80 durch die Steuersignal-Ausgabeeinheit 104 ausgegeben werden. Nur die zu einem Zeitpunkt validen Steuersignale werden ausgegeben.

Durch diese Anordnung wird gewährleistet, dass erkennbar negative Emotionen des Rollstuhlfahrers oder eine Unfähigkeit zur Steuerung des Rollstuhls 200 erfasst werden und der Sicherheitsmodus eingenommen wird. Zudem können Dritte sowie der Rollstuhlfahrer in der Folge nicht weiter körperlich geschädigt werden.

Bei Erfüllen der ersten Verlässlichkeitskriterien erfolgt die Ermittlung der ersten Steuersignale ST1 durch die Steuersignal-Ermittlungseinheit 102 des Rollstuhls 200 unter Verwendung der ersten Sensorsignale S1. Je nach Voreinstellung bedeutet ein Neigen des Kopfes des Rollstuhlfahrers nach oben ein Vorwärtsfahren oder Rückwärtsfahren, während das Neigen des Kopfs zur Seite eine Ausrichtung des Rollstuhls 200 auf diese Seite bedeutet.

In einem weiteren Ausführungsbeispiel des Rollstuhls 200 in Fig. 2 ist die Steuereinheit 100 ferner ausgebildet, neben den ersten Sensorsignalen S1 der an dem Wearable 10 angebrachten Trägheitsnavigationseinheit 11 unter Verwendung auch der zweiten Sensorsignale des bildbasierten Frontsensors 21 die ersten Steuersignale ST1 zu ermitteln. Dabei wird der Sicherheitsmodus eingenommen, wenn die ersten oder die zweiten Verlässlichkeitskriterien nicht erfüllt werden. Der Sicherheitsmodus wird auch eingenommen, wenn beide Verlässlichkeitskriterien nicht erfüllt werden. In diesem Ausführungsbeispiel basieren die zweiten Verlässlichkeitskriterien auf Genauigkeits- und Zuverlässigkeitsanalyse der ersten Sensorsignale S1 und der zweiten Sensorsignale S2 durch die Verlässlichkeitskriterien-Prüfeinheit 103.

Die Genauigkeits- und Zuverlässigkeitsanalyse wird unter Verwendung der ersten Sensorsignale S1 und der zweiten Sensorsignale S2, welche Merkmalswerte der Lage und der Rotation des Kopfs des Rollstuhlfahrers umfassen, ausgeführt. Anhand deren Abgleich wird die Zuverlässigkeit der Trägheitsnavigationseinheit 11 und des bildbasierten Frontsensors 21 ermittelt. Wenn die für die Steuerung des Rollstuhls 200 verwendeten Merkmale Lage und Rotation innerhalb vordefinierter Toleranzen übereinstimmen, werden für das Ermitteln der ersten Steuersignale ST1 die Merkmalswerte der Trägheitsnavigationseinheit 11 verwendet. Die Steuerung des Rollstuhls 200 erfolgt wie im vorangehenden Ausführungsbeispiel dargestellt.

Sollten eine oder mehrere der Toleranzen überschritten werden, wird nochmals auf die zweiten Sensorsignale S2 des bildbasierten Frontsensors 21 zurückgegriffen und bei schlechter Genauigkeit und Zuverlässigkeit, beispielsweise aufgrund von schlechter Bildqualität, ein Signal über die schlechte Zuverlässigkeit dem Rollstuhlfahrer auf dessen Mobiltelefon 20 übermittelt und dort dargestellt. Die schlechte Bildqualität kann dadurch erkannt werden, dass der für die Erkennung der Emotion des Rollstuhlfahrers verwendete Algorithmus nur jeweils geringe Wahrscheinlichkeiten den einzelnen Emotionen, z. B. Glückszustand, Unzufriedenheit zuordnet. Das Generieren von ersten Steuersignalen ST1 basierend auf den ersten Sensorsignalen S1 wird weiterhin ermöglicht.

Bei ausreichender Zuverlässigkeit des Frontsensors 21 wird der Sicherheitsmodus eingeleitet, denn die Verlässlichkeitskriterien-Prüfeinheit 103 nimmt einen Fehler der Trägheitsnavigationseinheit 11 an. Der Rollstuhl 200 wird angehalten.

Durch den fortwährenden Abgleich der Merkmalswerte der Trägheitsnavigationseinheit 11 und des bildbasierten Frontsensors 21 wird ein sicherer Betrieb des Rollstuhls 200 ermöglicht und es werden unverzüglich Maßnahmen zur Minimierung von Schadensfolgen bei Sensorfehlern eingeleitet.

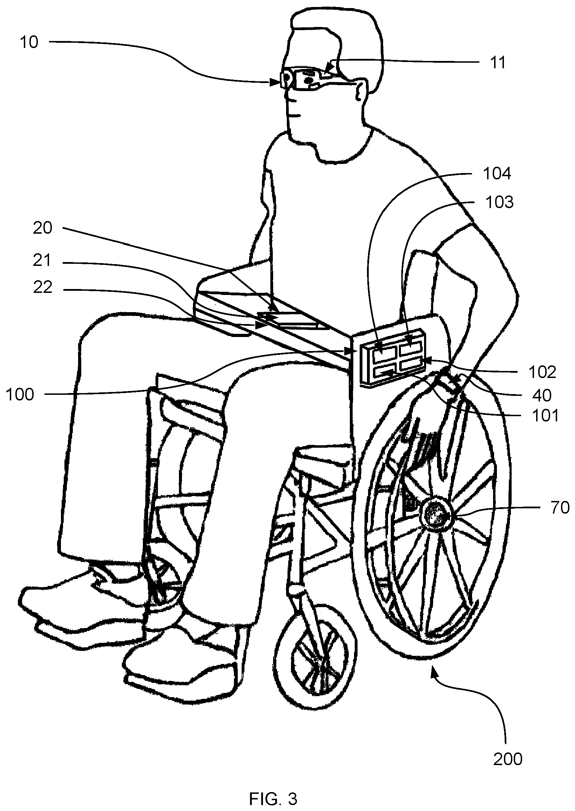

Fig. 3 zeigt ein weiteres Ausführungsbeispiel in der zudem als dritter Sensor ein Vitalitätsdatensensor 40 am Handgelenk des Rollstuhlfahrers angeordnet ist, um die Herzfrequenz des Rollstuhlfahrers zu erfassen und dritte Sensorsignale auszugeben. Die Steuereinheit 100 empfängt die z. B. über eine Bluetooth-Verbindung übertragenen dritten Sensorsignale mittels der Sensorsignal-Empfangseinheit 101, wobei die Verlässlichkeitskriterien-Prüfeinheit 103 bei Nichteinhalten eines zulässigen Herzfrequenzen-Wertebereichs durch die dritten Sensorsignale zudem prüft, ob mit hoher Wahrscheinlichkeit ein Besorgniszustand oder Angstzustand des Rollstuhlfahrers vorliegt. Ist auch dies der Fall, werden die zweiten Verlässlichkeitskriterien als nicht erfüllt bewertet. In der Folge wird der Sicherheitsmodus eingenommen. Die zweiten Steuersignale ST2 bewirken ein Anhalten des Rollstuhls 200.

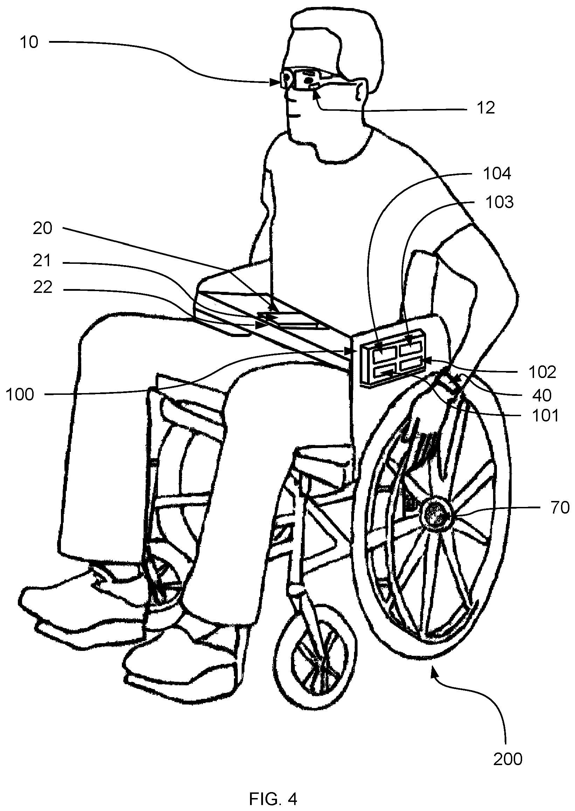

Fig. 4 zeigt ein weiteres Ausführungsbeispiel, welches ähnlich dem vorangehenden Beispiel ist. Jedoch weist dieses anstelle der Trägheitsnavigationseinheit 11 als ersten Sensor eine Augenmerkmalerfassungseinheit 12 auf. Diese ist an dem Wearable 10 angebracht und mit diesem, beispielsweise über eine Verkabelung, verbunden und ausgebildet Position und Translation der Iris eines Auges des Rollstuhlfahrers zu erfassen und erste Sensorsignale S1 auszugeben, wobei die Sensorsignal-Empfangseinheit 101 die ersten Sensorsignale S1 nach Weiterleitung durch das Wearable 10 empfängt und unter deren Verwendung die ersten Steuersignale ST1 zur Steuerung des Rollstuhls 200 ermittelt.

Anhand der Genauigkeits- und Zuverlässigkeitsanalyse der ersten Sensorsignale S1 der Augenmerkmalerfassungseinheit 12 und der zweiten Sensorsignale S2 des bildbasierten Frontsensors 21 wird durch die Verlässlichkeitskriterien-Prüfeinheit 103 überprüft, ob die zweiten Verlässlichkeitskriterien erfüllt sind. Die Verlässlichkeitskriterien-Prüfeinheit 103 stellt fest, ob Abweichungen der Merkmalswerte Position und Translation der Iris eines Auges vorliegen.

Abweichungen dieser Merkmalswerte müssen innerhalb vordefinierter, zulässiger Toleranzen der zweiten Verlässlichkeitskriterien liegen.

Sollten eine oder mehrere Toleranzen überschritten werden, wird wie in dem vorangehenden Ausführungsbeispiel auf die Sensorsignale des bildbasierten Frontsensors 21 zurückgegriffen und bei schlechter Genauigkeit und Zuverlässigkeit, beispielsweise aufgrund von schlechter Bildqualität, ein Signal über die schlechte Zuverlässigkeit dem Rollstuhlfahrer durch das Wearable 10 in sein Sichtfeld projiziert. Zudem wird die Information durch die Steuersignal-Ausgabeeinheit 104, z. B. über eine Bluetooth-Verbindung, auf sein Mobiltelefon 20 übermittelt. Das Generieren von ersten Steuersignalen ST1 basierend auf den ersten Sensorsignalen S1 wird weiterhin ermöglicht.

Bei nicht eingehaltener Toleranz, d. h. wenn die erlaubte Positionsabweichung der von beiden Sensoren ermittelten Position der Iris trotz ausreichender Zuverlässigkeit des bildbasierten Frontsensors 21 nicht eingehalten wird, wird der Sicherheitsmodus eingeleitet, da die Verlässlichkeitskriterien-Prüfeinheit 103 Fehler in der Augenmerkmalerfassungseinheit 12 annimmt. Der Sicherheitsmodus führt zum Anhalten des Rollstuhls 200.

Wenn die vom zweiten und vierten Sensor bestimmten Irispositionen und Translationen eines Auges innerhalb der Toleranzen übereinstimmen, wird von der Steuersignal-Ermittlungseinheit 102 ein finaler Positionswert durch Kalibrierung des Augen-Bewegungsbereichs ermittelt. Dabei wird die maximale Bewegung der Augen nach links, rechts, oben und unten ermittelt, um dadurch eine augenpositionsproportionale Steuerung des Rollstuhls 200 zu ermöglichen. Wenn dabei eine zuverlässige Irisposition nicht ermittelt werden kann, werden die zweiten Verlässlichkeitskriterien nicht erfüllt, der Sicherheitsmodus wird eingenommen und der Rollstuhl 200 wird angehalten.

Bei Nichtverletzen der ersten und zweiten Verlässlichkeitskriterien wird das augenmerkmalbasierte Steuern des Rollstuhls 200 durch Einnehmen eines Initialzustands, z. B. durch Fixieren eines Punktes in der Mitte des Augenpaares, gestartet. Auch kann eine Abfolge von Gesten, beispielsweise Augenblinzeln oder ein vordefiniertes Augenbewegungsmuster, das Starten der augenmerkmalbasierten Steuerung durch die Steuersignal-Ermittlungseinheit 102 bedeuten. Ein Augenbewegungsmuster ist dabei eine vom Rollstuhlfahrer definierte und ausgeführte Abfolge von Blickrichtungen.

Die Steuerung des Rollstuhls 200 erfolgt durch Zuordnung einer Blickrichtung zur gewünschten Bewegungsrichtung. Die Steuerung der Bewegungsrichtung des Rollstuhls 200 erfolgt, indem ein Blick nach links eine Rotation des Rollstuhls 200 nach links und ein Blick nach rechts eine Rotation des Rollstuhls nach rechts bedeutet. Der Rollstuhl 200 wird angehalten, indem eine vordefinierte Geste, beispielsweise das Schließen der Augen, oder ein mehrmaliges Augenblinzeln, vom ersten Sensor, der Augenmerkmalerfassungseinheit 12, oder vom zweiten Sensor, dem bildbasierten Frontsensor 21, erfasst wird.

Durch die fortwährende Analyse der Sensoren wird ein sicherer Betrieb des Rollstuhls 200 ermöglicht und es werden unverzüglich Maßnahmen zur Minimierung von Schadensfolgen bei Sensorfehlern eingeleitet. Die Sicherheit wird weiterhin durch den Vitalitätsdatensensor 40 erhöht. Denn sofern die dritten Sensorsignale einen Vitalitätsparameter-Wertebereich nicht erfüllen, werden die zweiten Verlässlichkeitskriterien nicht erfüllt. Der Sicherheitsmodus wird eingenommen.

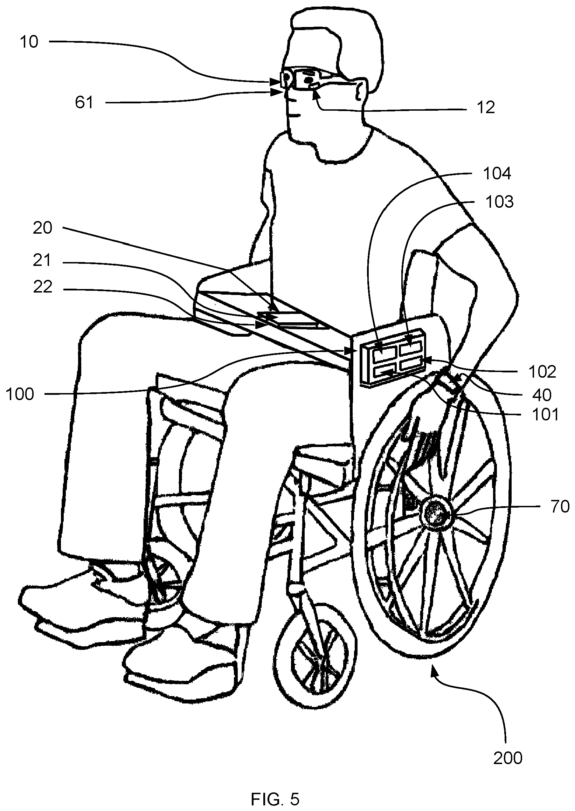

Fig. 5 zeigt ein weiteres, modifiziertes Ausführungsbeispiel in welchem der erste Sensor ein Wearable-Spracheingabesensor 61 ist, der an dem Wearable 10 angebracht ist und mit diesem z. B. durch eine Verkabelung, verbunden ist. Der Wearable-Spracheingabesensor 61 ist ausgebildet, erste Sensorsignale S1 auszugeben, wobei die Sensorsignal-Empfangseinheit 101 die ersten Sensorsignale S1 nach Weiterleitung durch das Wearable 10 empfängt und unter deren Verwendung die ersten Steuersignale ST1 zur Steuerung des Fahrzeugs 200 in der Steuersignal-Ermittlungseinheit 102 ermittelt.

Die Steuersignal-Ermittlungseinheit 102 ist dazu ausgebildet, bei der Verarbeitung der ersten Sensorsignale S1 zum Ermitteln der ersten Steuersignale ST1 eine Implementierung eines rekurrierenden neuronalen Netzes (RNN) zu verwenden. Diese Algorithmen zur Analyse abgeschlossener, sequentieller Sprach-Sensorsignale erlauben ein etwa gegenüber Fully-Connected-Neuronalen Netzen (FCN) zeitlich effizienteres Training sowie eine bei vergleichbarem Rechenaufwand verbesserte Erkennung der Spracheingaben des Rollstuhlfahrers.

Insbesondere für Rollstuhlfahrer mit eingeschränkten Sprachfähigkeiten erlauben die selbstlernenden neuronalen Netze eine Anpassung der Spracherkennung an die Stimme des Rollstuhlfahrers. Die durch Spracheingabe abgegebenen Steuerbefehle können einerseits in den ersten Steuersignalen ST1 zum (An-) Fahren oder Stoppen des Rollstuhls 200 führen. Andererseits werden Spracheingaben zum Bedienen einer Menüführung verwendet, welche dem Rollstuhlfahrer von dem Wearable 10 durch Projektion eines gewöhnlichen Head-up-Bildschirms angezeigt wird. Dadurch können Befehle, beispielsweise zum Öffnen von Türen, Bestellen von Aufzügen, Drücken von Knöpfen, Bedienen von Schaltern abgegeben werden.

Die Steuereinheit 100 ist ferner ausgebildet, neben den ersten Sensorsignalen S1 des Wearable-Spracheingabesensors 61 unter Verwendung auch zweiter Sensorsignale S2 des bildbasierten Frontsensors 21, die ersten Steuersignale ST1 zu ermitteln. Dabei ist der zweite Sensor auf den Kopf des Rollstuhlfahrers gerichtet und ausgebildet, Position und Translation beider Augen bzw. deren Iris zu erfassen und die zweiten Sensorsignale S2 auszugeben.

Als dritter Sensor wird der Vitalitätsdatensensor 40 am Handgelenk des Rollstuhlfahrers zur Bestimmung der Herzfrequenz angeordnet, wobei dieser sowie die Steuereinheit 100 dazu ausgebildet sind, die Funktionalitäten gemäß den vorangegangenen Ausführungsbeispielen bereitzustellen.

Als vierter Sensor wird die Augenmerkmalerfassungseinheit 12, angebracht am Wearable 10, verwendet. Die Augenmerkmalerfassungseinheit 12 ist wie der zweite Sensor, der bildbasierte Frontsensor 21 ausgebildet, die Position bzw. Translation eines Auges bzw. Iris des Rollstuhlfahrers, zu erfassen und vierte Sensorsignale auszugeben, wobei die Sensorsignal-Empfangseinheit 101 die vierten Steuersignale empfängt und unter deren Verwendung die Steuersignal-Ermittlungseinheit 102 die ersten Steuersignale ST1 zur Steuerung des Fahrzeugs 200 ermittelt. Bei der Augenmerkmalerfassungseinheit 12 handelt es sich um eine infrarotlichtbasierte Augenmerkmalerfassungseinheit.

Folglich werden in diesem Ausführungsbeispiel die ersten Sensorsignale S1, die zweiten Sensorsignale S2 und die vierten Sensorsignale für die Ermittlung der ersten Steuersignale ST1 verwendet. Dadurch ergibt sich ein redundantes System, wobei die Verwendung der infrarotlichtbasierten Augenmerkmalerfassungseinheit 12 zu einer diversitären Redundanz bei der Augenmerkmalerfassung durch den zweiten und vierten Sensor führt.

Der Sicherheitsmodus wird in diesem Ausführungsbeispiel eingenommen, wenn die Verlässlichkeitskriterien-Prüfeinheit 103 bestimmt, dass die ersten oder die zweiten Verlässlichkeitskriterien nicht erfüllt werden. Die Erfüllung der ersten Verlässlichkeitskriterien wird in der Verlässlichkeitskriterien-Prüfeinheit 103 zumindest unter Verwendung der Basisdaten des zweiten Sensors, des bildbasierten Frontsensors 21, wie in den vorangehenden Ausführungsbeispielen dargestellt, ermittelt.

Die Bewertung der zweiten Verlässlichkeitskriterien umfasst, wie in vorangegangenen Ausführungsbeispielen, eine Zuverlässigkeits- und Genauigkeitsanalyse. Basierend auf den zweiten Sensorsignalen S2 des bildbasierten Frontsensors 21 und den vierten Sensorsignalen der Augenmerkmalerfassungseinheit 12, überprüft die Verlässlichkeitskriterien-Prüfeinheit 103, ob die Abweichung der Position oder Translation eines Auges des Rollstuhlfahrers innerhalb der jeweils zulässigen Toleranz liegt.

Sollten eine oder mehrere der Toleranzen überschritten werden, wird, wie in den vorangehenden Ausführungsbeispielen beschrieben, auf die Sensorsignale des bildbasierten Frontsensors 21 zurückgegriffen und bei schlechter Genauigkeit und Zuverlässigkeit, beispielsweise aufgrund von schlechter Bildqualität, ein Signal über die schlechte Zuverlässigkeit dem Rollstuhlfahrer über das Wearable 10 oder auf sein Mobiltelefon 20 übermittelt. Das Generieren von ersten Steuersignalen ST1 unter Verwendung der vierten Sensorsignale durch die Steuersignal-Ermittlungseinheit 102 wird weiterhin ermöglicht und die ersten Steuersignale ST1 werden durch die Steuersignal-Ausgabeeinheit 104 ausgegeben.

Sofern bei Überschreiten einer oder mehrerer der Toleranzen keine geringe Zuverlässigkeit des bildbasierten Frontsensors 21 festgestellt wird, wird der Sicherheitsmodus eingenommen und der Rollstuhl 200 angehalten. Der Grund dafür ist, dass bei ausreichender Zuverlässigkeit des bildbasierten Frontsensors 21 die Verlässlichkeitskriterien-Prüfeinheit 103 annimmt, dass ein Fehler der Augenmerkmalerfassungseinheit 12 vorliegt.

Die Steuerung des Rollstuhls erfolgt wie im vorangehenden Ausführungsbeispiel. Zudem kann jedoch der erste Sensor, der Wearable-Spracheingabesensor 61, zur Erfassung eines Startbefehls verwendet werden.

Auch wird der Rollstuhl 200 durch Spracheingabe unter Verwendung des ersten Sensors, des Wearable-Spracheingabesensors 61, zum Anhalten gebracht.

Durch die fortwährende Analyse der Sensoren wird ein sicherer Betrieb des Rollstuhls 200 ermöglicht und es werden unverzüglich Maßnahmen zur Minimierung von Schadensfolgen bei Sensorfehlern eingeleitet. Die Sicherheit wird zudem durch die Verwendung des dritten Sensors, des Vitalitätsdatensensors 40, erhöht, denn sofern die dritten Sensorsignale einen Vitalitätsparameter-Wertebereich nicht erfüllen, bestimmt die Verlässlichkeitskriterien-Prüfeinheit 103, dass die zweiten Verlässlichkeitskriterien nicht erfüllt sind. Der Sicherheitsmodus wird gemäß den Bedingungen der vorangehenden Ausführungsbeispiele eingenommen.

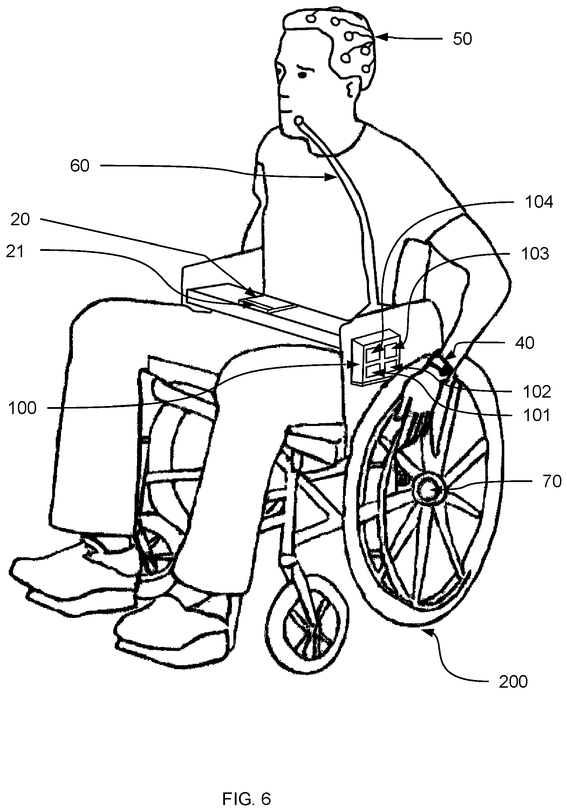

Fig. 6 zeigt ein weiteres, erfindungsgemäßes Ausführungsbeispiel des Rollstuhls 200, wobei anstelle des vierten Sensors, der Augenmerkmalerfassungseinheit 12, ein fünfter Sensor, eine Gehirn-Steuereinheit-Schnittstelle bzw. ein Eingabegerät 50 am Kopf des Rollstuhlfahrers angeordnet ist. Diese ist ausgebildet, fünfte Sensorsignale auszugeben, wobei die Sensorsignal-Empfangseinheit 101 die fünften Sensorsignale z. B. über eine Bluetooth-Verbindung empfängt und unter deren Verwendung die ersten Steuersignale ST1 zur Steuerung des Rollstuhls 200 in der Steuersignal-Ermittlungseinheit 102 ermittelt.

Eine Gehirn-Steuereinheit-Schnittstelle ist aus der US 2017/0042439A1 bekannt. Dabei wird um den Kopf einer Person herum ein Elektrodenband so angeordnet, dass Gehirnwellen gemessen und anschließend verarbeitet werden können. Dabei kann ein mentaler Zustand bzw. Emotion der Person ermittelt werden.

Die Gehirn-Steuereinheit-Schnittstelle bzw. das Eingabegerät 50 wird als primäre Steuerdatenquelle für die Generierung der ersten Steuersignale ST1 verwendet. Weiterhin werden anhand des bildbasierten Frontsensors 21 die ersten Verlässlichkeitskriterien ermittelt und bei Nichterfüllen der ersten Verlässlichkeitskriterien wird der Sicherheitsmodus eingenommen. Bei zusätzlich vom Vitalitätsdatensensor 40 erfasster, erhöhter Herzfrequenz nimmt die Verlässlichkeitskriterien-Prüfeinheit 103 einen kritischen körperlichen Zustand des Rollstuhlfahrers an und es werden zudem die zweiten Verlässlichkeitskriterien nicht erfüllt. Der Rollstuhl 200 wird angehalten.

In der Steuersignal-Ermittlungseinheit 102 werden die fünften Sensorsignale erfasst, sodass ein von dem fünften Sensor erfasster Gedanke des Rollstuhlfahrers einem gewünschten ersten Steuersignal ST1 zugeordnet wird.

Dazu kennt die Gehirn-Steuereinheit-Schnittstelle bzw. das Eingabegerät 50 vordefinierte Steuerbefehle, die als fünfte Sensorsignale ausgegeben werden, wenn der Rollstuhlfahrer an diese denkt.

Die fünften Sensorsignale umfassen jedoch nicht nur vordefinierte Steuerbefehle.

Als Teil der fünften Sensorsignale werden die vom fünften Sensor erfassten, abstrakten Gedankenmuster des Rollstuhlfahrers einem maschinellen Lernalgorithmus in der Steuersignal-Ermittlungseinheit 102 bereitgestellt. Der Lernalgorithmus lernt aus den Daten, sodass bei Erkennung eines bereits bekannten Gedankenmusters, welches einem Steuerbefehl des Rollstuhlfahrers zugeordnet werden kann, die ersten Steuersignale ST1 basierend auf dem erkannten Steuerbefehl ermittelt werden.

Der maschinelle Lernalgorithmus wird trainiert, indem gemäß den vorangehenden Ausführungsbeispielen die Emotion des Rollstuhlfahrers in Reaktion auf ein ermitteltes erstes Steuersignal ST1 aufgezeichnet und ebenfalls der Steuersignal-Ermittlungseinheit 102 bereitgestellt wird. Auch die Herzfrequenz des Rollstuhlfahrers wird bereitgestellt. Dadurch kann der maschinelle Lernalgorithmus erlernen, welche anhand der Gedankenmuster ermittelten Steuerbefehle vom Rollstuhlfahrer als angemessen bewertet werden. Dies ermöglicht eine Individualisierbarkeit der gedankenbasierten Steuerung des Rollstuhls 200 für den jeweiligen Rollstuhlfahrer.

Die Ermittlung der Emotionen des Rollstuhlfahrers ist jedoch in der Anwendung nicht auf das Training des maschinellen Lernalgorithmus beschränkt.

Weiterhin werden die fünften Sensorsignale auch genutzt, um festzustellen, ob der Rollstuhlfahrer in Panik geraten ist, beispielsweise, weil er sich in einem gefährlichen körperlichen Zustand befindet. Durch die Kombination mit dem ersten Sensor, dem Fahrzeug-Spracheingabesensor 60, dem zweiten Sensor, dem bildbasierten Frontsensor 21 sowie dem dritten Sensor dieses Ausführungsbeispiels, dem Vitalitätsdatensensor 40, können gefährliche Situationen leichter klassifiziert werden und das Fahren mit dem Rollstuhl 200 wird sicherer.

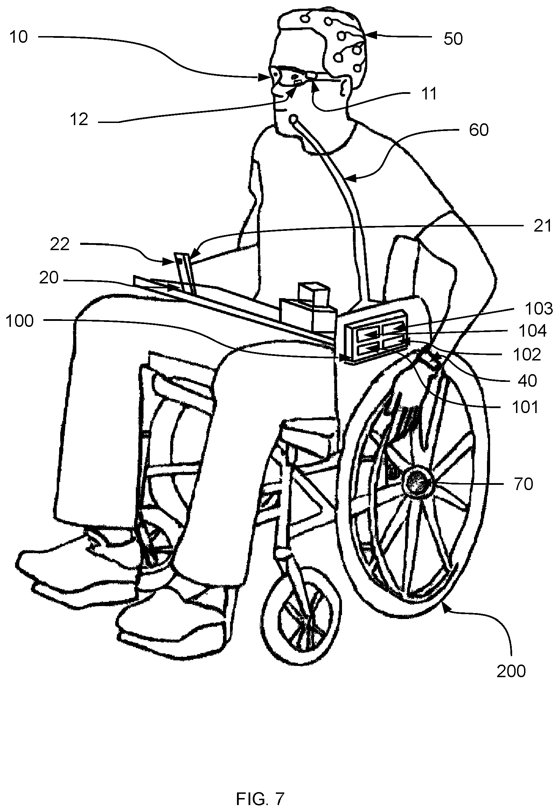

Fig. 7 zeigt ein weiteres Ausführungsbeispiel des Rollstuhls 200, wobei alle bisher verwendeten Sensoren zum Bereitstellen einer noch sichereren und zuverlässigeren Lösung kombiniert werden. Als erster Sensor wird die Trägheitsnavigationseinheit 11, angebracht am Wearable 10, verwendet, um die Lage und Rotation des Kopfs des Rollstuhlfahrers zu erfassen und erste Sensorsignale S1 an die Sensorsignal-Empfangseinheit 101 zu übermitteln und unter deren Verwendung die ersten Steuersignale ST1 in der Steuersignal-Ermittlungseinheit 102 zur Steuerung des Rollstuhls 200 zu ermitteln. Der zweite Sensor, der bildbasierte Frontsensor 21 erfasst den Kopf des Rollstuhlfahrers bzw. dessen Merkmale und übermittelt zweite Sensorsignale S2 der Steuereinheit-Empfangseinheit 101. Unter deren Verwendung prüft die Verlässlichkeitskriterien-Prüfeinheit 103, wie aus vorangehenden Ausführungsbeispielen bekannt, ob die ersten Verlässlichkeitskriterien erfüllt sind. Zudem werden unter Verwendung der zweiten Sensorsignale S2 die ersten Steuersignale ST1 zur Steuerung des Rollstuhls 200 ermittelt.

Der dritte Sensor, der Vitalitätsdatensensor 40, ist wie in vorangehenden Ausführungsbeispielen angeordnet und ausgebildet. Der vierte Sensor, eine Augenmerkmalerfassungseinheit 12, ist an dem Wearable 10 angebracht, und dazu ausgebildet, wie aus vorangehenden Ausführungsbeispielen bekannt, Position und Translation eines Auges bzw. einer Iris des Rollstuhlfahrers zu erfassen. Die vierten Sensorsignale werden der Steuereinheit 100 bereitgestellt, sodass unter deren Verwendung die ersten Steuersignale ST1 ermittelt werden können.

Basierend auf der Genauigkeits- und Zuverlässigkeitsanalyse der ersten Sensorsignale S1 der Trägheitsnavigationseinheit 11 und der zweiten Sensorsignale S2 des zweiten Sensors des bildbasierten Frontsensors 21, überprüft die Verlässlichkeitskriterien-Prüfeinheit 103, wie aus vorangehenden Ausführungsbeispielen bekannt, ob die Abweichungen der Lage oder der Rotation des Kopfs des Rollstuhlfahrers innerhalb der jeweils zulässigen Toleranzen liegen.

Bei der Genauigkeits- und Zuverlässigkeitsanalyse der zweiten Sensorsignale S2 des zweiten Sensors, des bildbasierten Frontsensors 21 und der vierten Sensorsignale des vierten Sensors, der Augenmerkmalerfassungseinheit 12, überprüft die Verlässlichkeitskriterien-Prüfeinheit 103, wie aus vorangehenden Ausführungsbeispielen bekannt, ob die Abweichung der Merkmale Position oder Translation eines Auges des Rollstuhlfahrers innerhalb der jeweiligen Toleranzen liegt.

Der fünfte Sensor, die Gehirn-Steuereinheit-Schnittstelle bzw. das Eingabegerät 50, ist wie aus dem vorangehenden Ausführungsbeispielen bekannt, angeordnet und ausgebildet. Die Steuersignal-Ermittlungseinheit 102 ermittelt unter Verwendung der übermittelten fünften Sensorsignale, Steuerbefehle des Rollstuhlfahrers zum Starten und Stoppen des Rollstuhls 200.

Als sechster Sensor wird der Fahrzeug-Spracheingabesensor 60 verwendet, der ausgebildet ist sechste Sensorsignale auszugeben, wobei die Steuereinheit 100 die sechsten Sensorsignale empfängt und unter deren Verwendung die ersten Steuersignale ST1 zur Steuerung des Rollstuhls 200 ermittelt. Durch den Fahrzeug-Spracheingabesensor 60 können Befehle zum Starten oder Stoppen des Rollstuhls 200 durch die sechsten Sensorsignale an die Sensorsignal-Empfangseinheit 101 übermittelt werden.

Sofern eine oder mehrere der Toleranzen nicht erfüllt werden, sind die zweiten Verlässlichkeitskriterien nicht erfüllt. Sollten die ersten oder zweiten Verlässlichkeitskriterien nicht erfüllt sein, nimmt die Vorrichtung 80 den Sicherheitsmodus ein. Sind sie erfüllt, können erste Steuersignale ST1 zur Bewegung des Rollstuhls 200 von der Steuersignal-Ausgabeeinheit 104 an die Fahrzeug-Aktuatoren 70 übermittelt werden.

Unter Verwendung der vierten Sensorsignale wird die gewünschte Fahrtrichtung durch die Steuersignal-Ermittlungseinheit 102 ermittelt und unter Verwendung der ersten Sensorsignale wird die gewünschte Geschwindigkeit in Fahrtrichtung ermittelt. Daraus werden die ersten Steuersignale ST1 ermittelt und von der Steuersignal-Ausgabeeinheit 104 an die Fahrzeug-Aktuatoren 70 ausgegeben.

Ein Nach-vorne-Blicken des Rollstuhlfahrers führt zu einer Fahrt nach vorne, das Blicken zu einer Seite führt zu einer Fahrt des Rollstuhls 200 in die jeweilige Richtung. Abhängig von der Voreinstellung führt eine Nickbewegung nach unten zu einer Erhöhung/Verringerung der Rollstuhlgeschwindigkeit, während eine Nickbewegung nach oben die jeweils entgegengesetzte Auswirkung auf die Rollstuhlgeschwindigkeit hat.

Dieses Ausführungsbeispiel hat gegenüber den Vorangegangenen zudem den Vorteil, dass die Sicherheit des Rollstuhlfahrers erhöht wird, da dieser in die Richtung der Fahrt blicken muss.

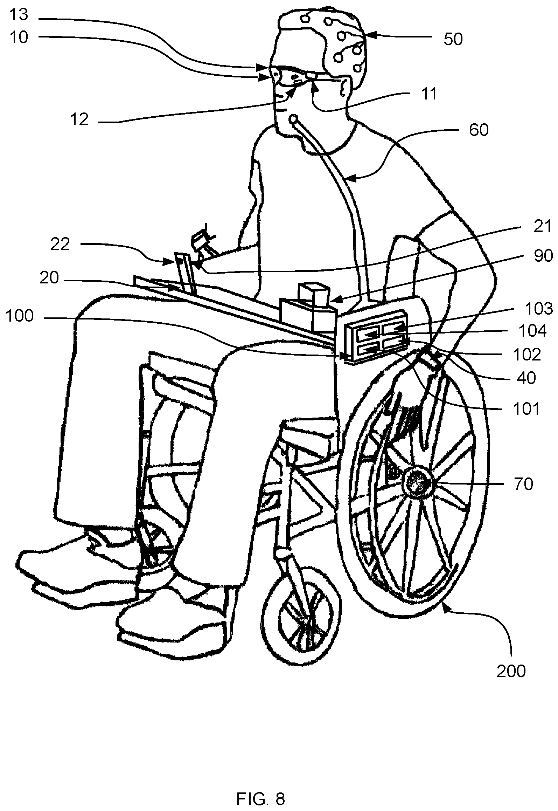

In Fig. 8 ist ein weiteres Ausführungsbeispiel abgebildet, das zudem eine erste umgebungserfassende Sensorbaugruppe 90, eine LIDAR-Sensorbaugruppe, umfasst. Diese ist am Rollstuhl 200 zum Erfassen der Umgebung angebracht und ausgebildet, erste Sensorbaugruppensignale auszugeben, wobei die Steuersignal-Erfassungseinheit 101 erste Sensorbaugruppensignale über eine Hochgeschwindigkeits-Datenleitung, beispielsweise eine Ethernet-Verbindung, empfängt und unter deren Verwendung die ersten Steuersignale ST1 ermittelt.

Die Wahrnehmung der Umgebung erfolgt zudem durch einen bildbasierten Rückseitensensor 22 des Mobiltelefons 20 und einen Wearable-Bildsensor 13, sodass unter deren Verwendung die ersten Sensorsignale ST1 von der Steuersignal-Ermittlungseinheit 102 ermittelt werden. Der Wearable-Bildsensor 13 ist z. B. über Datenleitungen mit dem Wearable 10 verbunden, sodass das Wearable 10 dessen Sensorsignale an die Sensorsignal-Empfangseinheit 102 übermittelt.

Dabei werden die erfassten Umgebungsinformationen von der Steuersignal-Ermittlungseinheit 102 und der Verlässlichkeitskriterien-Prüfeinheit 103 ausgewertet und bei kritischer Annäherung an Hindernisse, Gefälle, Steigungen oder Ähnlichem wird der Sicherheitsmodus eingenommen. Dieser führt zu einem Anhalten des Rollstuhls 200.

In einem weiteren Ausführungsbeispiel sind das Wearable 10 und das Mobiltelefon 20 ausgebildet, siebte Sensorsignale, insbesondere Passagierzieleingabe-Sensorsignale, auszugeben, wobei die Steuereinheit 100 zuvor verfügbare Zieleingabe-Auswahldaten dem Wearable 10 und dem Mobiltelefon 20 für die Passagierzieleingabe übermittelt hat.

Die Zieleingabe-Auswahldaten entstehen durch Auswertung der Sensorsignale der ersten umgebungserfassenden Sensorbaugruppe 90 durch die Steuereinheit 100. Bei den Zielen kann es sich grundsätzlich um alle Elemente handeln, deren Konturen von einer LIDAR-Sensorbaugruppe 90 in Kombination mit den weiteren umgebungserfassenden Sensoren 13, 22 erfasst werden können und die sich im Umfeld des Rollstuhlfahrers befinden.

Zieleingabe-Auswahldaten werden dem Rollstuhlfahrer, nach Vorbild eines Head-up-Bildschirms vom Wearable 10 in das Sichtfeld projiziert. Zudem werden die Informationen auf dem Bildschirm des Mobiltelefons 20 angezeigt. Die Sensorsignal-Empfangseinheit 101 empfängt die siebten Sensorsignale und ermittelt unter deren Verwendung die ersten Steuersignale ST1.

Dabei hat die Steuersignal-Ermittlungseinheit 102 die Aufgabe, eine Trajektorie zum Zielort zu berechnen, geeignete erste Steuersignale ST1 bereitzustellen sowie während der Fahrt zum Zielort basierend auf den Echtzeitdaten der umgebungserfassenden Sensoren 13, 22 und der LIDAR-Sensorbaugruppe 90 Anpassungen der Trajektorie, beispielsweise aufgrund der sich verändernden Umgebung, vorzunehmen, und angepasste erste Steuersignale ST1 bereitzustellen.

Bei Verwendung der LIDAR-Sensorbaugruppe 90 werden dreidimensionale, umgebungsabbildende Karten erzeugt. Den dreidimensionalen Punktewolken dieser Karten werden durch maschinelles Lernen Bildmerkmale aufgenommener Bilder der Umgebung zugeordnet. Dadurch ist es möglich, eine dreidimensionale Trajektorie zum Zielobjekt in der Umgebung zu generieren. Der Rollstuhl 200 bewegt sich entlang dieser Trajektorie, indem die generierten, ersten Steuersignale ST1 den Fahrzeug-Aktuatoren 70 bereitgestellt werden, um die Distanz zum Zielort bzw. -objekt zu minimieren. Wenn die minimale Distanz zum Ziel erreicht ist, hält der Rollstuhl 200 an.

Dem Rollstuhlfahrer werden in Echtzeit Zieleingabe-Auswahldaten in seinen Head-up-Bildschirm im Wearable 10 projiziert, indem der Wearable-Bildsensor 13 seine Sensorsignale der Steuereinheit 100 übermittelt und die umgebungserfassende LIDAR-Sensorbaugruppe 90 angeordnet ist, sich anhand der Blickrichtung des Rollstuhlfahrers auf die Umgebung auszurichten und erste Sensorbaugruppensignale der Steuereinheit 100 zu übermitteln. Die Steuereinheit ermittelt unter Verwendung vorgenannter Signale Zieleingabe-Auswahldaten und stellt diese dem Wearable 10 zum Projizieren in den Head-up-Bildschirm bereit.

Der Rollstuhlfahrer kann dadurch eine Zielobjektauswahl unmittelbar anhand der Echtzeit-Bilder bzw. eines Echtzeit-Videos durchführen und es werden siebte Sensorsignale, insbesondere Passagierzieleingabe-Sensorsignale, vom Wearable 10 an die Sensorsignal-Empfangseinheit 101 übermittelt. Die Steuersignal-Ermittlungseinheit 102 ermittelt unter Verwendung der siebten Sensorsignale die ersten Steuersignale ST1.

Die zweiten, dritten und fünften Sensorsignale werden von der Steuersignal-Ermittlungseinheit 102 verwendet, um Rückmeldung darüber zu geben, ob sich der Rollstuhl 200 aus Sicht des Rollstuhlfahrers auf dem richtigen Weg zum Ziel befindet. Dabei werden der Gesichtsausdruck des Rollstuhlfahrers, Vitalitätsparameter sowie die Gedanken des Rollstuhlfahrers überwacht, wobei der Sicherheitsmodus eingenommen wird, wenn anhand der Sensorsignale ein kritischer körperlicher Zustand des Rollstuhlfahrers erfasst wird. Auch kann jederzeit die automatisierte Ansteuerung eines ausgewählten Zielobjekts durch manuelle Übersteuerung durch den Rollstuhlfahrer, beispielsweise basierend auf den Sensorsignalen der Trägheitsnavigationseinheit 11, der Augenmerkmalerfassungseinheit 12 oder des bildbasierten Frontsensors 21, unterbrochen oder beendet werden.

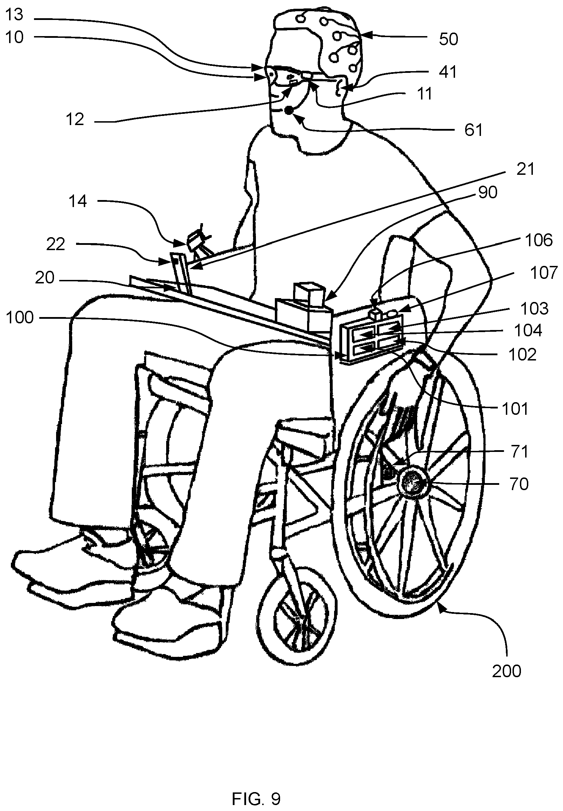

Fig. 9 zeigt ein weiteres erfindungsgemäßes Ausführungsbeispiel. Dabei wird ein achter Sensor, ein GPS-Positionssensor 106, am Rollstuhl 200 angeordnet und dieser ist ausgebildet, achte Sensorsignale auszugeben, wobei die Sensorsignal-Empfangseinheit 101 die achten Sensorsignale empfängt und unter deren Verwendung die ersten Steuersignale ST1 ermittelt.

Der Positionssensor 106 erlaubt die Darstellung einer Karte mit der aktuellen Position des Rollstuhls 200 bzw. des Rollstuhlfahrers mittels der Head-up-Projektion des Wearable 10 und auf sein Mobiltelefon 20. Die Verwendung einer Karte in Verbindung mit den dargestellten Zieleingabe-Auswahldaten erlaubt das Auswählen von Zielorten oder Objekten, die nicht von umgebungserfassenden Sensoren 13, 22 bzw. der umgebungserfassenden Sensorbaugruppe 90 wahrgenommen werden können. Die Auswahl eines Ziels erfolgt durch Führung eines virtuellen Mauszeigers per Augensteuerung in der Head-up-Projektion des Wearable 10 oder durch Auswahl auf dem Mobiltelefon 20.

Basierend auf der Zieleingabe des Rollstuhlfahrers werden die siebten Passagierzieleingabe-Sensorsignale der Steuersignal-Ermittlungseinheit 102 bereitgestellt. Unter Verwendung der Karte und der achten Sensorsignale des auf GPS-Signalen basierenden Positionssensors 106 wird eine Trajektorie zum Zielort bzw. -objekt bestimmt und entsprechende erste Steuersignale ST1 werden bereitgestellt.

Somit hat die Steuersignal-Ermittlungseinheit 102 die Aufgabe, eine Trajektorie zum Zielort zu berechnen, geeignete erste Steuersignale ST1 bereitzustellen, sowie während der Fahrt zum Zielort basierend auf den Echtzeitdaten der umgebungserfassenden Sensoren 13,22 und der umgebungserfassenden Sensorbaugruppe 90 Anpassungen der Trajektorie, beispielsweise aufgrund der sich verändernden Umgebung, vorzunehmen, und angepasste erste Steuersignale ST1 bereitzustellen. Nach Erreichen des Zielorts bzw. Zielobjekts hält der Rollstuhl 200 an.

Die zweiten, dritten und fünften Sensorsignale werden, wie in dem vorangegangenen Ausführungsbeispiel beschrieben, von der Steuersignal-Ermittlungseinheit 102 verwendet, um Rückmeldung darüber zu geben, ob sich der Rollstuhl 200 aus Sicht des Rollstuhlfahrers auf dem richtigen Weg zum Ziel befindet. Im Falle der Verletzung der aus vorangegangenen Ausführungsbeispielen bekannten ersten oder zweiten Verlässlichkeitskriterien wird der Sicherheitsmodus eingenommen.

Jederzeit kann die automatisierte Ansteuerung eines ausgewählten Zielobjekts durch manuelle Übersteuerung durch den Rollstuhlfahrer unterbrochen oder beendet werden.

Fig. 9 zeigt ein weiteres Ausführungsbeispiel, in dem zudem eine Fernkommunikationsvorrichtung 107 zur Übertragung von LTE- oder 5G-Signalen am Rollstuhl 200 angeordnet ist. Diese steht in Signalaustausch mit der Steuereinheit 100 und externen Mobilfunkanbietervorrichtungen. Dabei ist das Wearable 10 ebenfalls über eine LTE- oder 5G-Verbindung mit der Steuereinheit 100 verbunden, sodass eine (Video-)Kommunikation durchgeführt werden kann oder Bilder oder Videos, die von dem Wearable-Bildsensor 13 aufgenommen wurden, in persönlichen Nachrichten oder über soziale Netzwerke Anderen bereitgestellt werden können.

Des Weiteren kann im Fall eines eingeleiteten Sicherheitsmodus, beispielsweise aufgrund eines erkannten, kritischen körperlichen Zustands des Rollstuhlfahrers, mindestens ein Notfallkontakt automatisch kontaktiert werden. Dabei wird einerseits eine automatisierte Textnachricht übersendet. Außerdem wird ein Videoanruf gestartet.

Der Fig. 9 kann zudem ein weiteres Ausführungsbeispiel des Rollstuhls 200 entnommen werden, wobei als Fahrdynamik-Sensor 71, eine weitere Trägheitsnavigationseinheit mit integrierten Beschleunigungsmessern und Drehratenmessern und Kompass, angeordnet am Rollstuhl 200 und ausgebildet, neunte Sensorsignale auszugeben, verwendet wird. Die Sensorsignal-Empfangseinheit 101 empfängt die neunten Sensorsignale und ermittelt unter deren Verwendung die ersten Steuersignale ST1. Diese Sensorik erlaubt insbesondere die Regelung der Fahrdynamik durch Rückführung der Fahrdynamik-Sensorgrößen an die Steuersignal-Ermittlungseinheit 102, um gegenüber einer reinen Steuerung eine genauere Umsetzung der gewünschten Steuerungsbefehle zu ermöglichen.

In einem weiteren, der Fig. 9 entnehmbaren Ausführungsbeispiel, wird der bildbasierte Frontsensor 22 für die Erfassung des Gesichts des Rollstuhlfahrers durch einen bildbasierten Fahrzeug-Sensor 14 ersetzt, wobei dieser über eine Verbindung, z. B. USB-Verbindung, bildbasierte Sensorsignale der Steuersignal-Empfangseinheit 101 übermittelt und unter deren Verwendung die Steuersignal-Ermittlungseinheit 102 die ersten Steuersignale ST1 ermittelt sowie die Verlässlichkeitskriterien-Prüfeinheit 103 das Erfüllen der ersten Verlässlichkeitskriterien bewertet.

An dieser Stelle sei darauf hingewiesen, dass alle oben beschriebenen Teile, insbesondere die einzelnen Ausführungsformen und -beispiele, jeweils für sich - auch ohne im jeweiligen Zusammenhang zusätzlich beschriebener Merkmale, selbst wenn diese nicht explizit als optionale Merkmale im jeweiligen Zusammenhang individuell kenntlich gemacht worden sind, z. B. durch Verwendung von: insbesondere, vorzugsweise, beispielsweise, z. B., ggf., runden Klammern, etc. - und in Kombination oder jeglicher Unterkombination als eigenständige Ausgestaltungen bzw. Weiterbildungen der Erfindung, wie sie insbesondere in der Beschreibungseinleitung sowie den Ansprüchen definiert ist, anzusehen sind. Abweichungen hiervon sind möglich. Konkret sei darauf hingewiesen, dass das Wort "insbesondere" oder runde Klammern keine im jeweiligen Kontext zwingenden Merkmale kennzeichnen.

### Bezugszeichenliste

- 10: am Körper tragbares Computersystem (Wearable)
- 11: eine Trägheitsnavigationseinheit
- 12: Augenmerkmalerfassungseinheit
- 13: Wearable-Bildsensor
- 14: bildbasierter Fahrzeug-Sensor
- 20: Mobiltelefon
- 21: bildbasierter Frontsensor
- 22: bildbasierter Rückseitensensor
- 40: tragbarer Vitalitätsdatensensor
- 41: Ohr-Vitalitätsdatensensor
- 50: Gehirn-Steuereinheit-Schnittstelle bzw. Eingabegerät
- 60: Fahrzeug-Spracheingabesensor
- 61: Wearable-Spracheingabesensor
- 70: Fahrzeug-Aktuatoren
- 71: Fahrdynamik-Sensoren
- 80: Vorrichtung zum Navigieren und/oder Bahnführen und/oder Stabilisieren eines Fahrzeugs
- 90: Umgebungserfassende Sensorbaugruppe
- 100: Steuereinheit
- 101: Sensorsignal-Empfangseinheit
- 102: Steuersignal-Ermittlungseinheit
- 103: Verlässlichkeitskriterien-Prüfeinheit
- 104: Steuersignal-Ausgabeeinheit
- 106: Positionssensor
- 107: Fernkommunikationsvorrichtung
- 200: Fahrzeug, insbesondere Rollstuhl
- ST1: erste Steuersignale
- ST2: zweite Steuersignale
- S1: erste Sensorsignale
- S2: zweite Sensorsignale

## Patentansprüche

1. Vorrichtung zum Navigieren und/oder Bahnführen und/oder Stabilisieren eines Fahrzeuges (200), die Vorrichtung (80) umfassend:
- mindestens einen ersten Sensor, nämlich eine Trägheitsnavigationseinheit (11), der dazu ausgebildet und angeordnet ist, mindestens einen ersten Körperteil eines Passagiers des Fahrzeugs (200) zu erfassen und erste Sensorsignale (S1) auszugeben;
- mindestens einen zweiten Sensor, nämlich einen bildbasierten Sensor (21, 22), der dazu ausgebildet und angeordnet ist, mindestens den ersten Körperteil des Passagiers und/oder dessen Merkmale zu erfassen und zweite Sensorsignale (S2) auszugeben; und
- Steuereinheit (100), die dazu ausgebildet ist,
a) die ersten und zweiten Sensorsignale (S1, S2) zu empfangen;
b) basierend zumindest auf den ersten Sensorsignalen (S1) erste Steuersignale (ST1) zur Steuerung des Fahrzeugs (200) zu ermitteln;
c) basierend zumindest auf den zweiten Sensorsignalen (S2) zu ermitteln, ob die ersten Steuersignale (ST1) mindestens erste Verlässlichkeitskriterien erfüllen;
d) einen Sicherheitsmodus einzunehmen, wenn die Steuereinheit (100) bestimmt, dass die ersten Steuersignale (ST1) nicht mindestens die ersten Verlässlichkeitskriterien erfüllen.

2. Vorrichtung nach Anspruch 1,
**dadurch gekennzeichnet, dass**
der erste Körperteil der Kopf des Passagiers ist, wobei
a) anhand geometrischer Abmessungen und/oder Größenverhältnissen und/oder Merkmalen des Gesichts und/oder Gesten, die von mindestens dem zweiten Sensor erfasst werden, die Steuereinheit (100) Basisdaten ermittelt;
b) die ersten Verlässlichkeitskriterien unter Verwendung der Basisdaten ermittelt werden.

3. Vorrichtung nach Anspruch 1 oder 2,
**dadurch gekennzeichnet, dass**
die Steuereinheit (100) ferner ausgebildet ist, unter Verwendung mindestens der zweiten Sensorsignale die ersten Steuersignale (ST1) zu ermitteln; und
e) den Sicherheitsmodus einzunehmen, wenn die Steuereinheit (100) bestimmt, dass die ersten Steuersignale (ST1) nicht mindestens die ersten und zweite Verlässlichkeitskriterien erfüllen, wobei die ersten und/oder zweiten Verlässlichkeitskriterien durch Genauigkeits- und/oder Zuverlässigkeitsanalyse mindestens der ersten Sensorsignale (S1) und der zweiten Sensorsignale (S2) ermittelt werden.

4. Vorrichtung nach einem der vorangehenden Ansprüche,
**dadurch gekennzeichnet, dass**
die Steuereinheit das Erfüllen der ersten Verlässlichkeitskriterien durch Implementierung von maschinellem Lernen ermittelt.

5. Vorrichtung nach Anspruch 3,
**dadurch gekennzeichnet, dass**
ein Vitalitätsdatensensor an einem Körperteil oder zwischen zwei Körperteilen angeordnet ist und Vitalitätsparameter als charakterisierende Merkmale erfasst und dritte Sensorsignale ausgibt, wobei die Steuereinheit die dritten Sensorsignale empfängt und bei nicht Einhalten eines Vitalitätsparameter-Wertebereichs durch die dritten Sensorsignale die zweiten Verlässlichkeitskriterien nicht erfüllt werden.

6. Vorrichtung nach einem der vorangehenden Ansprüche,
**dadurch gekennzeichnet, dass**
mindestens eine erste Sensorbaugruppe (90) am Fahrzeug (200) zum Erfassen der Umgebung angebracht und ausgebildet ist, erste Sensorbaugruppensignale auszugeben, wobei die Steuereinheit (100) erste Sensorbaugruppensignale empfängt und unter deren Verwendung die ersten Steuersignale (ST1) ermittelt.

7. Vorrichtung nach einem der vor vorangehenden Ansprüche,
**dadurch gekennzeichnet, dass**
eine Fernkommunikationsvorrichtung (107) am Fahrzeug angeordnet und ausgebildet ist, einen Signalaustausch mit der Steuereinheit und Mobilfunkanbietervorrichtungen zum Durchführen von Videokommunikation und/oder Fern-Gesundheitszustandsüberwachung des Passagiers durchzuführen.

8. Vorrichtung nach einem der vorangehenden Ansprüche,
**dadurch gekennzeichnet, dass**
der erste Sensor an oder in einem Wearable (10) angebracht und dazu ausgebildet ist, das erste Körperteil oder deren Merkmale des Passagiers des Fahrzeugs (200) zu erfassen und erste Sensorsignale auszugeben, wobei unter deren Verwendung die ersten Steuersignale (ST1) zur Steuerung des Fahrzeugs (200) ermittelt werden.

9. Vorrichtung nach Anspruch 8,
**dadurch gekennzeichnet, dass**
ein vierter Sensor, beispielsweise eine Augenmerkmalerfassungseinheit (12), an oder in dem Wearable (10) angebracht und dazu ausgebildet ist, ein zweites Körperteil oder deren Merkmale des Passagiers des Fahrzeugs (200) zu erfassen und vierte Sensorsignale auszugeben, wobei unter deren Verwendung die ersten Steuersignale (ST1) zur Steuerung des Fahrzeugs (200) ermittelt werden.

10. Vorrichtung nach einem der vorangehenden Ansprüche,
**dadurch gekennzeichnet, dass**
ein fünfter Sensor, insbesondere eine Gehirn-Steuereinheit-Schnittstelle bzw. Eingabegerät (50) auf und/oder im Kopf des Passagiers angeordnet und ausgebildet ist, fünfte Sensorsignale auszugeben, wobei unter deren Verwendung die ersten Steuersignale (ST1) zur Steuerung des Fahrzeugs (200) ermittelt werden.

11. Vorrichtung nach dem der vorangehenden Ansprüche, insbesondere nach Anspruch 9,
**dadurch gekennzeichnet, dass**
ein Wearable-Spracheingabesensor (61) oder ein Fahrzeug-Spracheingabesensor (60) ausgebildet ist, sechste Sensorsignale auszugeben, wobei die Steuereinheit (100) die sechsten Sensorsignale empfängt und unter deren Verwendung die ersten Steuersignale (ST1) zur Steuerung des Fahrzeugs (200) ermittelt.

12. Vorrichtung nach einem der Ansprüche 9-11,
**dadurch gekennzeichnet, dass**
das Wearable (10) und/oder ein Smartphone (20) ausgebildet und angeordnet ist, siebte Sensorsignale, nämlich Passagierzieleingabe-Sensorsignale, auszugeben, wobei die Steuereinheit (100) verfügbare Zieleingabe-Auswahldaten dem Wearable (10) und/oder dem Smartphone (20) für die Passagierzieleingabe übermittelt sowie die siebten Sensorsignale empfängt und unter deren Verwendung die ersten Steuersignale (ST1) ermittelt.

13. Vorrichtung nach einem der vorangehenden Ansprüche,
**dadurch gekennzeichnet, dass**
ein achter Sensor, nämlich ein Positionssensor (106) angeordnet und ausgebildet ist, achte Sensorsignale auszugeben, wobei die Steuereinheit (100) achte Sensorsignale empfängt und unter deren Verwendung die ersten Steuersignale (ST1) ermittelt und/oder Fahrdynamik-Sensoren (71) am Fahrzeug (200) angeordnet und ausgebildet sind, neunte Sensorsignale auszugeben, wobei die Steuereinheit (100) die neunten Sensorsignale empfängt und unter deren Verwendung die ersten Steuersignale (ST1) ermittelt.

14. Fahrzeug, umfassend eine
- Vorrichtung (80) nach einem der vorangehenden Ansprüche;
- Fahrzeug-Aktuatoren (70), angeordnet zum Antreiben des Fahrzeugs (200) und ausgebildet zum Empfangen und Verarbeiten der Steuersignale der Vorrichtung (80).

15. Verfahren zum Steuern einer Vorrichtung zum Navigieren und/oder Bahnführen und/oder Stabilisieren eines Fahrzeugs (200), insbesondere nach Anspruch 14,
umfassend die Schritte:
a) Empfangen mindestens erster Sensorsignale (S1), nämlich trägheitsnavigationsbasierter Sensorsignale, welche mindestens ein erstes Körperteil eines Passagiers des Fahrzeugs abbilden; und Empfangen zweiter Sensorsignale (S1, S2) eines bildbasierten Frontsensors (21),
der dazu ausgebildet und angeordnet ist, mindestens das erste Körperteil oder dessen charakterisierende Merkmale des Passagiers des Fahrzeugs (200) abzubilden;
b) Ermitteln von ersten Steuersignalen (ST1) zur Steuerung des Fahrzeugs (200) basierend zumindest auf den ersten Sensorsignale (S1);
c) Bestimmen, ob die ersten Steuersignale (ST1) mindestens erste Verlässlichkeitskriterien basierend zumindest auf den zweiten Sensorsignalen (S2) erfüllen;
d) Einnehmen eines Sicherheitsmodus, wenn die Steuereinheit (100) bestimmt, dass die Steuersignale (ST1) nicht mindestens die ersten Verlässlichkeitskriterien erfüllen.

## Claims

1. A device for navigating and/or guiding the path and/or stabilizing a vehicle (200), the device (80) comprising:
- at least one first sensor, in particular inertial navigation unit (11), which is designed and arranged to detect at least one first body part of a passenger of the vehicle (200) and to output first sensor signals (S1);
- at least one second sensor, in particular image-based sensor (21, 22), which is designed and arranged to detect at least the first body part of the passenger and/or their features, and to output second sensor signals (S2); and
- control unit (100) designed to
a) receive the first and second sensor signals (S1, S2);
b) determine first control signals (ST1) for controlling the vehicle (200) based at least on the first sensor signals (S1);
c) determine whether the first control signals (ST1) meet at least first reliability criteria based at least on the second sensor signals (S2);
d) adopt a safety mode if the control unit (100) determines that the first control signals (ST1) do not meet at least the first reliability criteria.

2. The device of claim 1, **characterized in that** the first body part is the passenger's head, wherein
a) the control unit (100) determines basic data on the basis of geometric dimensions and/or size ratios and/or features of the face and/or gestures detected by at least the second sensor;
b) the first reliability criteria are determined using the basic data.

3. The device according to claim 1 or 2, **characterized in that** the control unit (100) is further designed to determine the first control signals (ST1) using at least the second sensor signals; and
e) to adopt the safety mode when the control unit (100) determines that first control signals (ST1) do not meet at least the first and/or second reliability criteria, wherein the first and/or second reliability criteria are determined by accuracy and/or reliability analysis of at least the first sensor signals (S1) and the second sensor signals (S2).

4. The device according to one of the preceding claims, **characterized in that** the control unit determines the fulfillment of the first reliability criteria by implementing machine learning.

5. The device according to claim 3, **characterized in that** a vitality data sensor is arranged on a body part or between two body parts and detects vitality parameters as characterizing features and outputs third sensor signals, wherein the control unit receives the third sensor signals and, if a vitality parameter value range is not met by the third sensor signals, the second reliability criteria are not met.

6. The device according to one of the preceding claims, **characterized in that** at least one first sensor assembly (90) is mounted on the vehicle (200) for sensing the environment and is designed to output first sensor assembly signals, wherein the control unit (100) receives first sensor assembly signals and determines the first control signals (ST1) using them.

7. The device according to one of the preceding claims, **characterized in that** a remote communication device (107) is arranged on the vehicle and designed to perform signal exchange with the control unit and mobile telephony provider devices for performing video communication and/or remote health condition monitoring of the passenger.

8. The device according to one of the preceding claims, **characterized in that** the first sensor is mounted on or in a wearable (10) and is designed to detect the first body part or its features of the passenger of the vehicle (200) and to output first sensor signals, wherein, by using said signals, the first control signals (ST1) for controlling the vehicle (200) are determined.

9. The device according to claim 8, **characterized in that** a fourth sensor, in particular an eye feature detection unit (12) is mounted on or in the wearable (10) and is designed to detect second body part or its features of the passenger of the vehicle (200) and to output fourth sensor signals, wherein, by using said signals, the first control signals (ST1) for controlling the vehicle are determined.

10. The device according to one of the preceding claims, **characterized in that** a first sensor, in particular a brain control unit interface or input device (50), is arranged on and/or in the head of the passenger and is designed to output fifth sensor signals, wherein, by using said signals, the first control signals (ST1) for controlling the vehicle (200) are determined.

11. The device according to one of the preceding claims, in particular according to claim 9, **characterized in that** a wearable speech input sensor (61) or a vehicle speech input sensor (60) is designed to output sixth sensor signals, wherein the control unit (100) receives the sixth sensor signals and, by using said signals, determines the first control signals (ST1) for controlling the vehicle (200).

12. The device according to one of claims 9-11, **characterized in that** the wearable (10) and/or a smartphone (20) is designed and arranged to output seventh sensor signals, in particular passenger destination input sensor signals, wherein the control unit (100) transmits available destination input selection data to the wearable (10) and/or the smartphone (20) for passenger destination input as well as receives the seventh sensor signals and, by using said signals, determines the first control signals (ST1).

13. The device according to one of the preceding claims, **characterized in that** an eighth sensor, in particular a position sensor (106) is arranged and designed to output eighth sensor signals, wherein the control unit (100) receives eighth sensor signals and, by using said signals, determines the first control signals (ST1) and/or vehicle dynamics sensors (71) are arranged on the vehicle (200) and are designed to output ninth sensor signals, wherein the control unit (100) receives the ninth sensor signals and, by using said signals, determines the first control signals (ST1).

14. A vehicle comprising a
- device (80) according to one of the preceding claims;
- vehicle actuators (70) arranged to drive the vehicle (200) and designed to receive and process the control signals of the device (80).

15. A method for controlling a device for navigating and/or guiding the path and/or stabilizing a vehicle (200), in particular according to claim 14, comprising the steps of
a) receiving at least first sensor signals (S1), in particular inertial navigation-based sensor signals, which describe at least a first body part of a passenger of the vehicle; and receiving second sensor signals (S1, S2) from an image-based front sensor (21), which is designed and arranged to describe at least the first body part or its characterizing features of the passenger of the vehicle (200);
b) determining first control signals (ST1) for controlling the vehicle (200) based at least on the first sensor signals (S1);
c) determining whether the first control signals (ST1) meet at least first reliability criteria based on at least the second sensor signals (S2);
d) adopting a safety mode when the control unit (100) determines that the control signals (ST1) do not meet at least the first reliability criteria.

## Revendications

1. Dispositif pour la navigation et/ou le guidage de trajectoire et/ou la stabilisation d'un véhicule (200), lequel dispositif (80) comprend :
- au moins un premier capteur, à savoir une unité de navigation à inertie (11), qui est conçu et disposé pour acquérir au moins une première partie du corps d'un passager du véhicule (200) et émettre des premiers signaux de capteur (51) ;
- au moins un deuxième capteur, à savoir un capteur d'image (21, 22), qui est conçu et disposé pour acquérir au moins la première partie du corps du passager et/ou ses caractéristiques et pour émettre des deuxièmes signaux de capteur (S2), et
- une unité de commande (100) conçue pour :
a) recevoir les premiers et deuxièmes signaux de capteur (S1, S2) ;
b) déterminer, sur la base au moins des premiers signaux de capteur (S1), des premiers signaux de commande (ST1) pour la commande du véhicule (200) ;
c) déterminer, sur la base au moins des deuxièmes signaux de capteur (S2), si les premiers signaux de commande (ST1) satisfont au moins des premiers critères de fiabilité ;
d) passer dans un mode de sécurité si l'unité de commande (100) détermine que les premiers signaux de commande (ST1) ne satisfont pas au moins les premiers critères de fiabilité.

2. Dispositif selon la revendication 1, **caractérisé en ce que** la première partie du corps est la tête du passager, dans lequel
a) l'unité de commande (100) détermine des données de base à partir de dimensions géométriques et/ou de rapports de dimensions et/ou de caractéristiques du visage et/ou de gestes acquis au moins par le deuxième capteur ;
b) les premiers critères de fiabilité sont déterminés en utilisant les données de base.

3. Dispositif selon la revendication 1 ou 2, **caractérisé en ce que** l'unité de commande (100) est en outre conçue pour déterminer les premiers signaux de commande (ST1) en utilisant au moins les deuxièmes signaux de capteur et pour
e) passer dans le mode de sécurité quand l'unité de commande (100) détermine que les premiers signaux de commande (ST1) ne satisfont pas au moins les premiers et deuxièmes critères de fiabilité, les premiers et/ou deuxièmes critères de fiabilité étant déterminés par l'analyse de l'exactitude et/ou de la fiabilité des premiers signaux de capteur (51) et des deuxièmes signaux de capteur (S2) au minimum.

4. Dispositif selon l'une des revendications précédentes, **caractérisé en ce que** l'unité de commande détermine que les premiers critères de fiabilité sont satisfaits en implémentant un apprentissage automatique.

5. Dispositif selon la revendication 3, **caractérisé en ce qu'**un capteur de données vitales est disposé sur une partie du corps ou entre deux parties du corps et acquiert des paramètres vitaux comme caractéristiques et émet des troisièmes signaux de capteur, l'unité de commande recevant les troisièmes signaux de capteurs et les deuxièmes critères de fiabilité n'étant pas satisfaits si les troisièmes signaux de capteur ne se situent pas dans une plage de valeurs des paramètres vitaux.

6. Dispositif selon l'une des revendications précédentes, **caractérisé en ce qu'**au moins un premier module de capteur (90) est placé sur le véhicule (200) pour acquérir l'environnement et conçu pour émettre des premiers signaux de module de capteur, l'unité de commande (100) recevant des premiers signaux de module de capteur et les utilisant pour déterminer les premiers signaux de commande (ST1).

7. Dispositif selon l'une des revendications précédentes, **caractérisé en ce qu'**un dispositif de télécommunication (107) est disposé sur le véhicule et conçu pour exécuter un échange de signaux avec l'unité de commande et avec des dispositifs d'opérateurs de radiotéléphonie pour réaliser une communication en vidéo et/ou une télésurveillance de l'état de santé du passager.

8. Dispositif selon l'une des revendications précédentes, **caractérisé en ce que** le premier capteur est placé sur un objet personnel connecté (10) et conçu pour acquérir la première partie du corps du passager du véhicule (200) ou des caractéristiques de celle-ci et émettre des premiers signaux de capteur, les premiers signaux de commande (ST1) pour la commande du véhicule (200) étant déterminés en utilisant ceux-ci.

9. Dispositif selon la revendication 8, **caractérisé en ce qu'**un quatrième capteur, par exemple une unité d'acquisition de caractéristiques des yeux (12), est placée sur ou dans l'objet personnel connecté (10) et conçue pour acquérir une deuxième partie du corps du passager du véhicule (200) ou des caractéristiques de celle-ci et pour émettre des quatrièmes signaux de capteur, les premiers signaux de commande (ST1) pour la commande du véhicule (200) étant déterminés en utilisant ceux-ci.

10. Dispositif selon l'une des revendications précédentes, **caractérisé en ce qu'**un cinquième capteur, en particulier une interface entre le cerveau et l'unité de commande ou un appareil de saisie (50), est disposé sur et/ou dans la tête du passager et conçu pour émettre des cinquièmes signaux de capteur, les premiers signaux de commande (ST1) pour la commande du véhicule (200) étant déterminés en utilisant ceux-ci.

11. Dispositif selon l'une des revendications précédentes, en particulier selon la revendication 9, **caractérisé en ce qu'**un capteur d'entrée vocale de l'objet personnel connecté (61) ou un capteur d'entrée vocale du véhicule (60) est conçu pour émettre des sixièmes signaux de capteur, l'unité de commande (100) recevant les sixièmes signaux de capteur et déterminant, en utilisant ceux-ci, les premiers signaux de commande (ST1) pour la commande du véhicule (200).

12. Dispositif selon l'une des revendications 9 à 11, **caractérisé en ce que** l'objet personnel connecté (10) et/ou un smartphone (20) est conçu et disposé de façon à émettre des septièmes signaux de capteur, à savoir des signaux de capteur de saisie de la destination du passager, l'unité de commande (100) transmettant des données de sélection de saisie de la destination à l'objet personnel connecté (10) et/ou au smartphone (20) pour la saisie de la destination du passager et recevant les septièmes signaux de capteur pour déterminer à l'aide de ceux-ci les premiers signaux de commande (ST1).

13. Dispositif selon l'une des revendications précédentes, **caractérisé en ce qu'**un huitième capteur, à savoir un capteur de position (106), est disposé et conçu de façon à émettre des huitièmes signaux de capteur, l'unité de commande (100) recevant des huitièmes signaux de capteur et déterminant à l'aide de ceux-ci les premiers signaux de commande (ST1) et/ou des capteurs de dynamique de marche (71) sont disposés sur le véhicule (200) et conçus pour émettre des neuvièmes signaux de capteur, l'unité de commande (100) recevant les neuvièmes signaux de capteur et les utilisant pour déterminer les premiers signaux de commande (ST1).

14. Véhicule comprenant
- un dispositif (80) selon l'une des revendications précédentes ;
- des actionneurs de véhicule (70), disposés pour entraîner le véhicule (200) et conçus pour recevoir et traiter les signaux de commande du dispositif (80).

15. Procédé pour commander un dispositif pour la navigation et/ou le guidage de trajectoire et/ou la stabilisation d'un véhicule (200), en particulier selon la revendication 14, comprenant les étapes suivantes :
a) réception d'au moins des premiers signaux de capteur (S1), à savoir des signaux de capteur d'une navigation à inertie qui représentent au moins une première partie du corps d'un passager du véhicule, et
réception de deuxièmes signaux de capteur (S1, S2) d'un capteur d'image frontal (21) qui est conçu et disposé pour représenter au moins la première partie du corps ou ses caractéristiques du passager du véhicule (200) ;
b) détermination de premiers signaux de commande (ST1) pour la commande du véhicule (200) sur la base au moins des premiers signaux de capteur (S1) ;
c) détermination de la satisfaction au moins de premiers critères de fiabilité par les premiers signaux de commande (ST1) sur la base au moins des deuxièmes signaux de capteur (S2) ;
d) passage dans un mode de sécurité si l'unité de commande (100) détermine que les signaux de commande (ST1) ne satisfont pas au moins les premiers critères de fiabilité.
